# EUROPEAN PATENT SPECIFICATION

(11) **EP 0 936 923 B1**
(45) Date of publication and mention of the grant of the patent: **17.12.2003**
(21) Application number: 97912367.6
(22) Date of filing: 17.11.1997
(51) Int. Cl.: A61K 39/395

(54) **SUPPRESSION OF TNFalpha AND IL-12 IN THERAPY**
UNTERDRÜCKUNG VON TNFalpha UND IL-12 IN DER THERAPIE
SUPPRESSION DU FNTalpha ET DE IL-12 EN THERAPIE

(30) Priority: 15.11.1996 US 749979
(43) Date of publication of application: 25.08.1999
(73) Proprietor: KENNEDY INSTITUTE OF RHEUMATOLOGY, Hammersmith, London W6 8LH (GB)
(72) Inventor: FELDMANN, Marc, London N6 4QT (GB); MALFAIT, Anne-Marie Aline Michel, London SW13 0NS (GB); BUTLER, Debra Maree, Wallington, Surrey SM6 7NB (GB); BRENNAN, Fionula Mary, London NW11 0RL (GB); MAINI, Ravinder Nath, London SW13 9EW (GB)
(74) Representative: Kirkham, Nicholas Andrew
(86) International application number: GB9703151
(87) International publication number: WO98022137

(56) References cited:
- WO-A-92/16553
- WO-A-94/08619
- WO-A-95/24918
- MALFAIT A M ET AL: "Prolonged blockage of IL - 12 attenuates collagen-induced arthritis (CIA) but short-term blockage can exacerbate the disease." 60TH NATIONAL SCIENTIFIC MEETING OF THE AMERICAN COLLEGE OF RHEUMATOLOGY AND THE 31ST NATIONAL SCIENTIFIC MEETING OF THE ASSOCIATION OF RHEUMATOLOGY HEALTH PROFESSIONALS, OCTOBER 18-22, 1996. ARTHRITIS & RHEUMATISM 39 (9 SUPPL.) S63, XP002055279
- MAURI C ET AL: "Th1-Th2 response in CIA: Dominant role of IFN-gamma and IL - 12 in the induction of arthritis." 60TH NATIONAL SCIENTIFIC MEETING OF THE AMERICAN COLLEGE OF RHEUMATOLOGY AND THE 31ST NATIONAL SCIENTIFIC MEETING OF THE ASSOCIATION OF RHEUMATOLOGY HEALTH PROFESSIONALS, OCTOBER 18-22, 1996. ARTHRITIS & RHEUMATISM 39 (9 SUPPL.) S247, XP002055280

## Description

### BACKGROUND OF THE INVENTION

Monocytes and macrophages secrete cytokines known as tumor necrosis factor alpha (TNFα), interleukin-1 (IL-1) and interleukin-6 (IL-6) in response to endotoxin or other stimuli. TNFα is a soluble homotrimer of 17 kD protein subunits (Smith *et al., J. Biol. Chem. 262*:6951-6954 (1987)). A membrane-bound 26 kD precursor form of TNF also exists (Kriegler *et al., Cell 53*:45-53 (1988)). For reviews of TNF, see Beutler *et al., Nature 320*:584 (1986); Old, Science 230:630 (1986) ; and Le *et al., Lab. Invest*. 56: 234 (1987).

Cells other than monocytes or macrophages also produce TNFα. For example, human non-monocytic tumor cell lines produce TNFα (Rubin *et al., J. Exp. Med. 164*:1350 (1986); Spriggs *et al., Proc. Natl. Acad. Sci. USA 84*:6563 (1987)). CD4+ and CD8+ peripheral blood T lymphocytes and some cultured T and B cell lines (Cuturi *et al., J. Exp. Med. 165*:1581 (1987) ; Sung *et al., J. Exp. Med. 168*:1539 (1988); Turner *et al., Eur. J. Immunol. 17*:1807-1814 (1987)) also produce TNFα.

TNFα causes pro-inflammatory actions which result in tissue injury, such as degradation of cartilage and bone (Saklatvala, *Nature 322*:547-549 (1986); Bertolini, *Nature 319*:516-518 (1986)), induction of adhesion molecules, inducing procoagulant activity on vascular endothelial cells (Pober *et al., J. Immunol. 136*:1680 (1986)), increasing the adherence of neutrophils and lymphocytes (Pober *et al., J. Immunol. 138*:3319 (1987)), and stimulating the release of platelet activating factor from macrophages, neutrophils and vascular endothelial cells (Camussi *et al., J. Exp. Med. 166*:1390 (1987)).

Recent evidence associates TNFα with infections (Cerami *et al., Immunol. Today 9*:28 (1988)), immune disorders, neoplastic pathologies (Oliff *et al., Cell 50*:555 (1987)), autoimmune pathologies and graft-versus-host pathologies (Piguet *et al., J. Exp. Med. 166*:1280 (1987)). The association of TNFα with cancer and infectious pathologies is often related to the host's catabolic state. Cancer patients suffer from weight loss, usually associated with anorexia.

The extensive wasting which is associated with cancer, and other diseases, is known as "cachexia" (Kern *et al., J*. *Parent. Enter. Nutr. 12*:286-298 (1988)). Cachexia includes progressive weight loss, anorexia, and persistent erosion of lean body mass in response to a malignant growth. The cachectic state causes much cancer morbidity and mortality. There is evidence that TNFα is involved in cachexia in cancer, infectious pathology, and other catabolic states (see, e.g., Beutler and Cerami, *Ann. Rev. Immunol*. *7*:625-655 (1989)).

TNFα is believed to play a central role in gram-negative sepsis and endotoxic shock (Michie *et al*., *Br. J. Surg. 76*:670-671 (1989); Debets *et al., Second Vienna Shock Forum,* p. 463-466 (1989); Simpson *et al., Crit. Care Clin. 5*:27-47 (1989)), including fever, malaise, anorexia, and cachexia. Endotoxin strongly activates monocyte/macrophage production and secretion of TNFα and other cytokines (Kornbluth *et al., J. Immunol*. *137*:2585-2591 (1986)). TNFα and other monocyte-derived cytokines mediate the metabolic and neurohormonal responses to endotoxin (Michie *et al., New Engl. J. Med*. *318*:1481-1486 (1988)). Endotoxin administration to human volunteers produces acute illness with flu-like symptoms including fever, tachycardia, increased metabolic rate and stress hormone release (Revhaug *et al., Arch. Surg. 123*:162-170 (1988)). Circulating TNFα increases in patients suffering from Gram-negative sepsis (Waage *et al., Lancet 1*:355-357 (1987); Hammerle *et al., Second Vienna Shock Forum,* p. 715-718 (1989); Debets *et al., Crit. Care Med. 17*:489-497 (1989); Calandra *et al., J. Infect. Dis. 161*:982-987 (1990)).

Thus, TNFα has been implicated in inflammatory diseases, autoimmune diseases, viral, bacterial and parasitic infections, malignancies, and/or neurogenerative diseases and is a useful target for specific biological therapy in diseases, such as rheumatoid arthritis and Crohn's disease. Beneficial effects in open-label trials with a chimeric monoclonal antibody to TNFα (cA2) have been reported with suppression of inflammation and with successful retreatment after relapse in rheumatoid arthritis (Elliott *et al*., Arthritis Rheum. 36:1681-1690 (1993); and Elliott *et al*., *Lancet 344*:1125-1127 (1994)) and in Crohn's disease (Van Dullemen *et al*., *Gastroenterology 109*:129-135 (1995)). Beneficial results in a randomized, double-blind, placebo-controlled trial with cA2 have also been reported in rheumatoid arthritis with suppression of inflammation (Elliott *et al., Lancet 344*:1105-1110 (1994)).

Monocytes, macrophages and other antigen-presenting cells, such as dendritic cells, also secrete a cytokine known as interleukin-12 (IL-12) in response to bacterial products and immunological stimuli. IL-12 is a heterodimeric cytokine, consisting of a p40 and a p35 subunit, with potent immunoregulatory properties (reviewed in Trinchieri, *Annu. Rev. Immunol. 13*:251-276 (1995); and Trinchieri, Blood 84:4008-4027 (1994)). IL-12 enhances natural killer (NK) -mediated cytotoxicity and induces interferon γ (IFN-γ) production by NK cells and T lymphocytes (Wolf *et al., J. Immunol. 146*:3074-3081 (1991); and Chan *et al., J. Exp. Med. 173*:869-879 (1991)). IL-12 plays a key role in promoting T helper type 1 (Th1) immune responses *in vitro* (Manetti *et al*., *J. Exp. Med, 177*:1199-1204 (1993)) and *in vivo* (Sypek et *al., J. Exp. Med. 177*:1797-1802 (1993); and Heinzel et *al., J. Exp. Med. 177*:1505-1509 (1993)).

IL-12 has been shown to accelerate the onset of autoimmune diabetes, a Th1-mediated disease, in nonobese diabetic (NOD) mice (Trembleau *et al., J. Exp. Med*. *181*:817-821 (1995)). It has also been demonstrated that IL-12 can replace Mycobacterium tuberculosis when immunizing DBA/1 mice with type II collagen in oil to profoundly upregulate a Th1-type autoimmune response, resulting in arthritis (Germann *et al., Proc. Natl*. *Acad. Sci. USA 92*:4823-4827 (1995)).

Antibodies against IL-12 have been shown to be beneficial in experimental models for autoimmune diseases that are Th1 driven, such as experimental allergic encephalomyelitis (EAE) (Leonard *et al., J. Exp. Med. 181*:381-386 (1995); and Leonard *et al*., International Publication No. WO 9524918 (September 21, 1995)) and 2,4,6-trinitrobenzene sulfonic acid (TNBS)-induced chronic intestinal inflammation in mice, a model for human inflammatory bowel disease (Neurath *et al., J*. *Exp. Med. 182*:1281-1290 (1995)). However, paradoxical effects of IL-12 in experimental models have been described. For example, very high doses of IL-12 administered in the induction phase of type II collagen induced arthritis (CIA) have been reported to suppress the immune response and prevent the onset of arthritis (Hess *et al., Eur. J. Immunol. 26*:187-191 (1996)). Similarly, although continuous administration of IL-12 accelerates the onset of diabetes in NOD mice, intermittent administration completely prevents the diabetes (Trembleau *et al., Immunol. Today 16*: 383-387 (1995), and Leonard *et al*., International Publication No. WO 9524918 (September 21, 1995)).

WO-A-9524918 is directed to a method of treating autoimmune conditions comprising administering to a mammalian subject lL-12 or an lL-12 antagonist.

60th National Scientific meeting of the American College of Rheumatology and the 31st National Scientific meeting of the Association of Rheumatology Health Professionals, October 18-22, 1996. Arthritis & Rheumatism 39 (9 Suppl.), S63; Malfait A M et al "Prolonged blockage of lL-12 attenuates collagen-induced arthritis (CIA) but short term blockage can exacerbate the disease".

Shows that lL-12 is of critical importance in the induction of collagen-induced arthritis by mediating a Th1 response to CII but suggests a bimodal role for this cytokine in the disease process.

The role of Th1/Th2-type responses in the development of CIA has been investigated by measuring IPN-γ and IL-4/IL-10 production in type II collagen (CII)-stimulated draining lymph node cells cultured at different stages of the disease (Mauri *et al., Eur. J. Imm. 26*:1511-1518 (1996)). It was found that IFN-γ production dramatically increased at the time of disease onset and subsequently declined throughout the disease and the remission phase, in favor of the Th2 cytokines IL-4 and IL-10.

The association between Th1 CD4+ T cell responses. and the onset of arthritis suggests that IL-12 blockade should be beneficial in CIA. Indeed, IL-12p40 knockout mice displayed a dramatic decrease in severity of CIA. In addition, systemic administration of IL-12 in mice immunized with type II collagen in incomplete Freund's adjuvant (IFA) was found to promote the development of a Th1 response and the subsequent manifestations of arthritis (Mauri *et al., Arth. Rheum. 39*(9 Suppl.) ;S247 (1996)).

However, in adult male DBA/1 mice, the effects of neutralizing IL-12 with anti-IL-12 antibodies in the induction phase of the disease were dependent on the treatment protocol (Malfait *et al., Arth. Rheum*. *39*(9 Suppl.) :S63 (1996)). Prolonged blockade of IL-12 from immunization until the onset of disease dramatically attenuated the severity of the arthritis. However, when IL-12 was blocked immediately after immunization for only a short period (i.e. 2 weeks), a dichotomous response was observed. Half of the mice developed a very mild arthritis or no disease, the other half developed an unusually severe arthritis. Neutralizing anti-IL-12 antibodies administered after disease onset had minimal, if any, effect on the course of CIA.

60th National Scientific meeting of the American College of Rheumatology and the 31st National Scientific meeting of the Association of Rheumatology Health Professionals, October 18-22, 1996. Arthritis & Rheumatism 39 (9 Suppl.), S247; Mauri C et al "Th1-Th2 response in CIA: Dominant role of IFN-gamma and IL-12 in the induction of arthritis".

Shows that systemic administration of IFNγ or lL-12 in mice immunized with collagen in IFA promotes the development of a Th1 response and the subsequent manifestation of arthritis.

### SUMMARY OF THE INVENTION

The present invention is based on the unexpected discovery that co-administration of a TNFα antagonist and a IL-12 antagonist produces a rapid and sustained reduction in the signs and symptoms associated with TNF-mediated diseases, As a result of Applicants' invention, a method is provided herein for treating and/or preventing a TNFα-mediated disease in an individual comprising co-administering a TNFα antagonist and an IL-12 antagonist to the individual in therapeutically effective amounts. The present invention may further to be used in a method for treating and/or preventing recurrence of a TNFα-mediated disease in an individual comprising co-administering a TNFα antagonist and an IL-12 antagonist to the individual in therapeutically effective amounts. TNFα-mediated diseases include rheumatoid arthritis, Crohn's disease, and acute and chronic immune diseases associated with an allogenic transplantation (e.g., renal, cardiac, bone marrow, liver, pancreatic, small intestine, skin or lung transplantation).

Therefore, in one embodiment, the invention relates to use in a method of treating and/or preventing (such as preventing relapse of) rheumatoid arthritis in an individual comprising co-administering a TNF antagonist and an IL-12 antagonist to the individual in therapeutically effective amounts. In a second embodiment, the invention relates to use in a method of treating and/or preventing (such as preventing relapse of) Crohn's disease in an individual comprising co-administering a TNF antagonist and an IL-12 antagonist to the individual in therapeutically effective amounts. In a third embodiment, the invention relates to use in a method of treating and/or preventing acute or chronic immune disease associated with a transplantation in an individual comprising co-administering a TNF antagonist and an IL-12 antagonist to the individual in therapeutically effective amounts.

A further embodiment of the invention relates to compositions comprising a TNF antagonist and an IL-12 antagonist.

TNF antagonists useful in the methods and compositions of the present invention include anti-TNF antibodies, antigen-binding fragments thereof, and receptor molecules which bind specifically to TNF; compounds which prevent and/or inhibit TNF synthesis, TNF release or its action on target cells, such as thalidomide, tenidap, phosphodiesterase inhibitors (e.g, pentoxifylline and rolipram), A2b adenosine receptor agonists and A2b adenosine receptor enhancers; compounds which prevent and/or inhibit TNF receptor signalling, such as mitogen activated protein (MAP) kinase inhibitors; compounds which block and/or inhibit membrane TNF cleavage, such as metalloproteinase inhibitors; compounds which block and/or inhibit TNF activity, such as angiotensin converting enzyme (ACE) inhibitors (e.g., captopril); and compounds which block and/or inhibit TNF production and/or synthesis, such as MAP kinase inhibitors.

IL-12 antagonists useful in the methods and compositions of the present invention include anti-IL-12 antibodies and antigen-binding fragments thereof; IL-12 p40 homodimers; IL-12 p35 homodimers; receptor molecules which bind specifically to IL-12; compounds (e.g., drugs and other agents, including antibodies) which decrease and/or block IL-12 production and/or synthesis, such as β-adrenergic agonists (e.g., salbutamol); and compounds which block and/or interfere with IL-12 receptor signalling. IL-12 antagonists useful in the present invention also include agents which are antagonists of signals that drive IL-12 production and/or synthesis, such as agents which decrease and/or block CD40 or its ligand.

In a particular embodiment of the invention, an inflammatory mediator other than a TNF antagonist and/or an IL-12 antagonist can be used in addition to the TNF antagonist and/or the IL-12 antagonist.

### BRIEF DESCRIPTION OF THE DRAWINGS

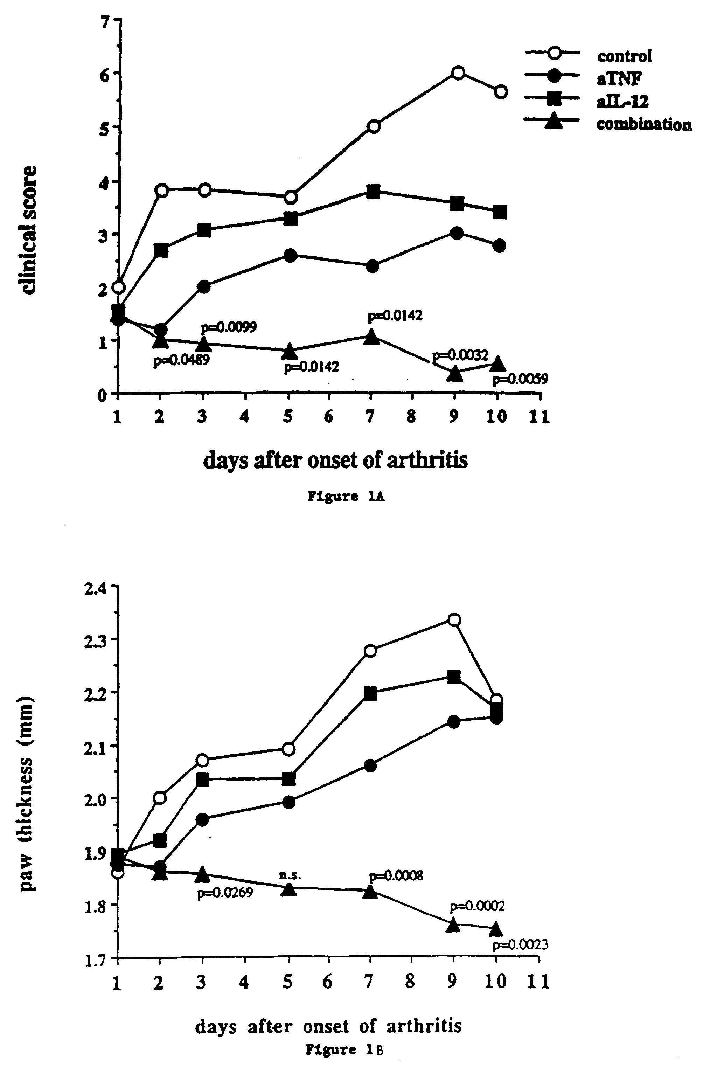

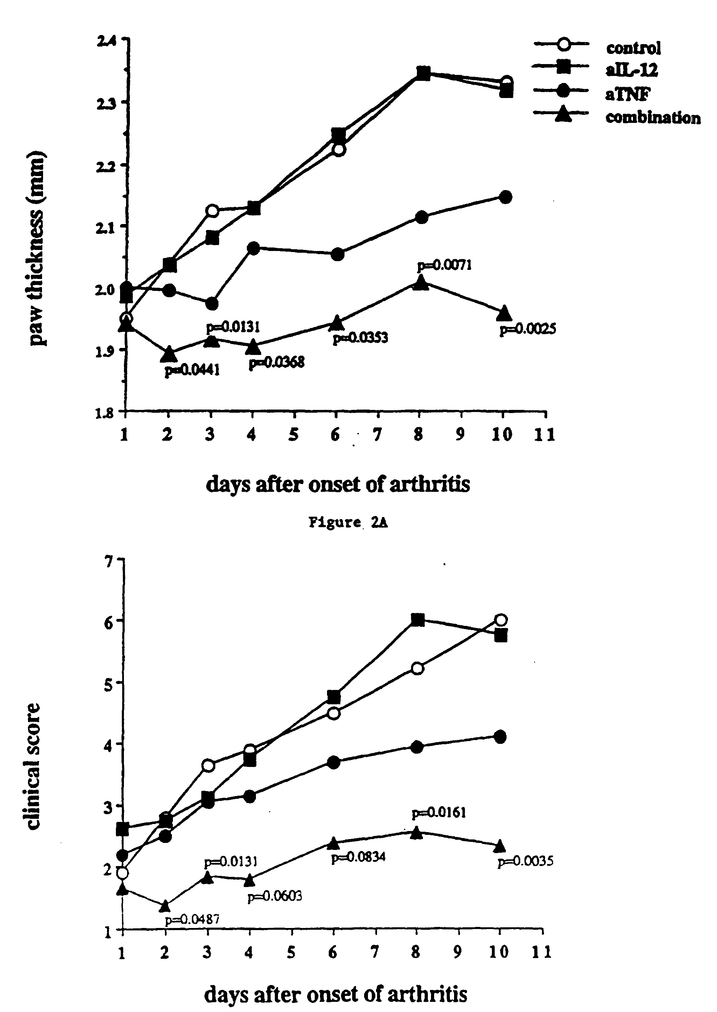

Figures 1A-1B and 2A-2B are graphs showing the effect of administering anti-TNF antibody, anti-IL-12 antibody, or a combination of anti-TNF antibody and anti-IL-12 antibody to male DBA/1 mice with induced arthritis on the suppression of arthritis as assessed by paw-swelling measurements (Figures 1B and 2A) and clinical score (Figures 1A and 2B). White circle = PBS control; black circle = anti-TNF antibody; square = anti-IL-12 antibody; triangle = anti-TNF antibody plus anti-IL-12 antibody.

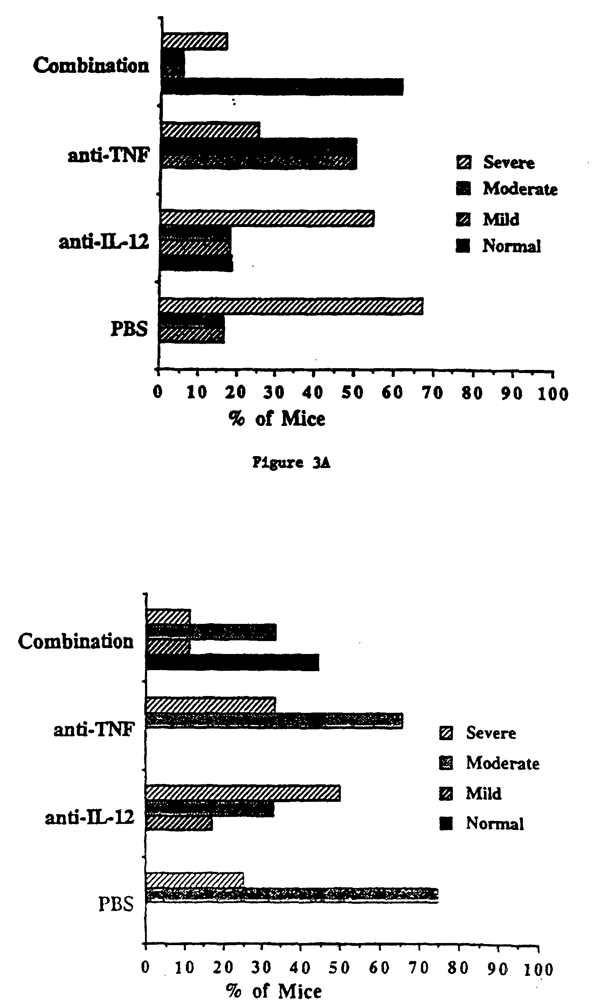

Figures 3A and 3B are bar graphs showing the results of a histological analysis of arthritis within the hind paw (Figure 3A) and knee (Figure 3B) of male DBA/1 mice with induced arthritis after treatment with anti-TNF antibody, anti-IL-12 antibody, or a combination of anti-TNF antibody and anti-IL-12 antibody.

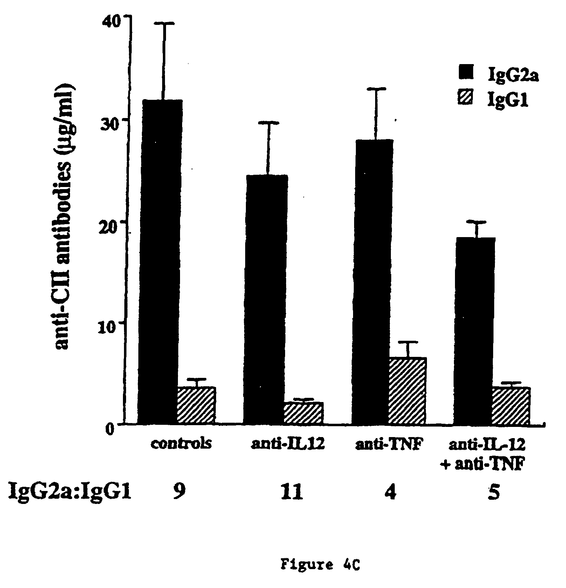

Figures 4A-4C are bar graphs showing serum levels of anti-type II collagen antibodies in male DBA/1 mice with induced arthritis after treatment with anti-TNF antibody, anti-IL-12 antibody, or a combination of anti-TNF antibody and anti-IL-12 antibody. Error bars represent 1 SD.

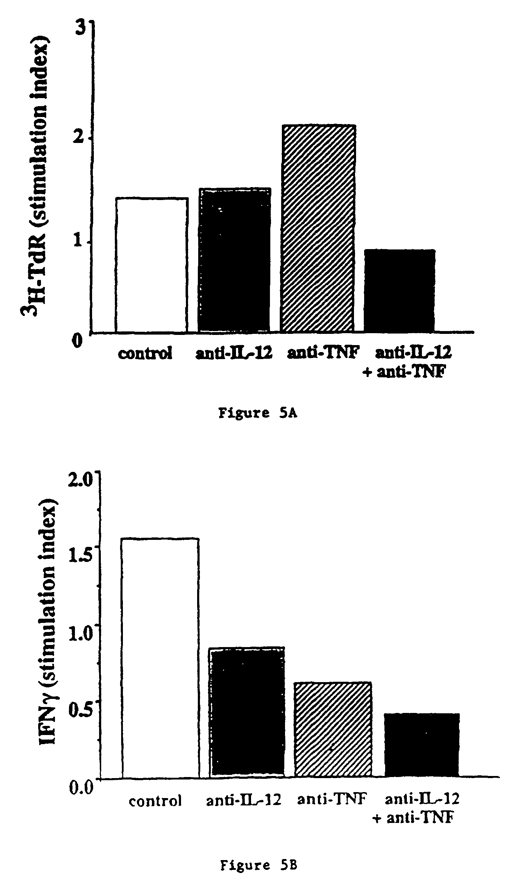

Figures 5A and 5B are bar graphs showing antigen-specific T-cell proliferation (Figure 5A) and IFNγ production (Figure 5B) in lymph node cells from male DBA/1 mice with induced arthritis after treatment with anti-TNF antibody, anti-IL-12 antibody, or a combination of anti-TNF antibody and anti-IL-12 antibody.

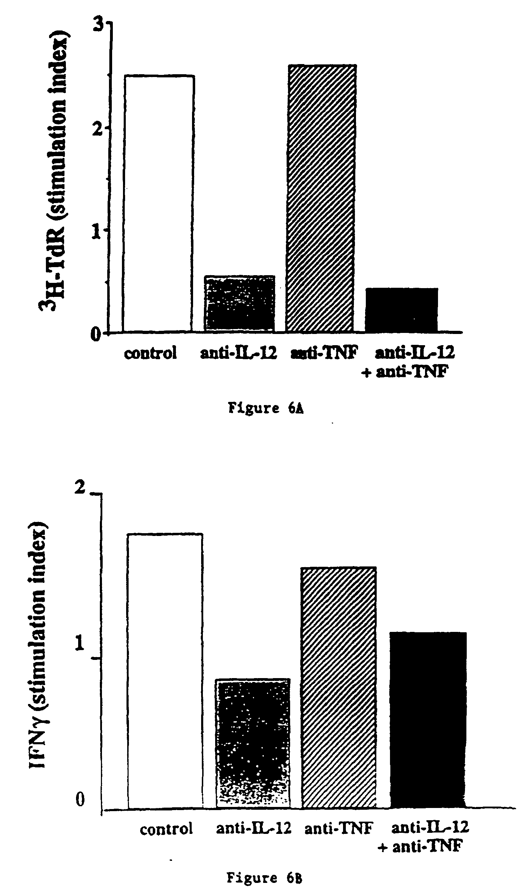

Figures 6A and 6B are bar graphs showing the in vitro effects of anti-IL-12 antibody, anti-TNF antibody and a combination of anti-IL-12 antibody and anti-TNF antibody on antigen-specific T-cell proliferation (Figure 6A) and IFNγ production (Figure 6B) in lymph node cells from male DBA/1 mice with induced arthritis.

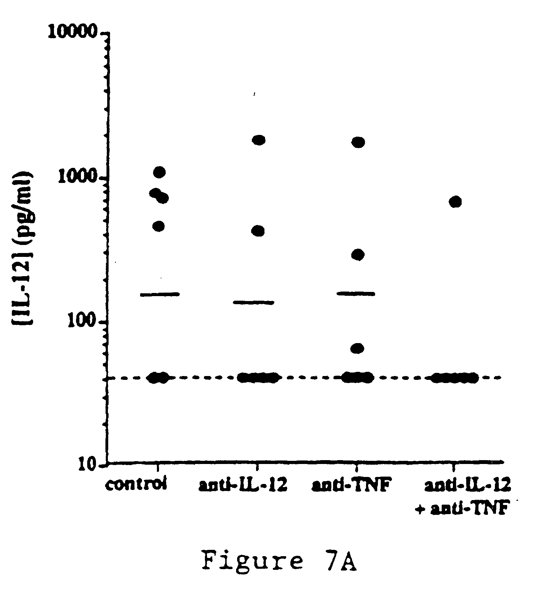

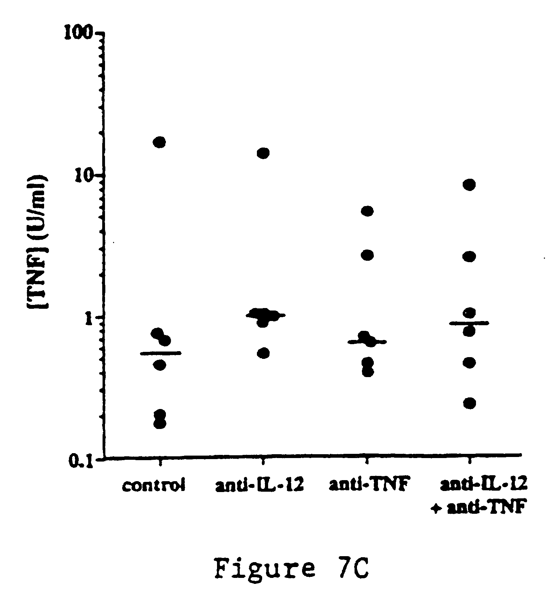

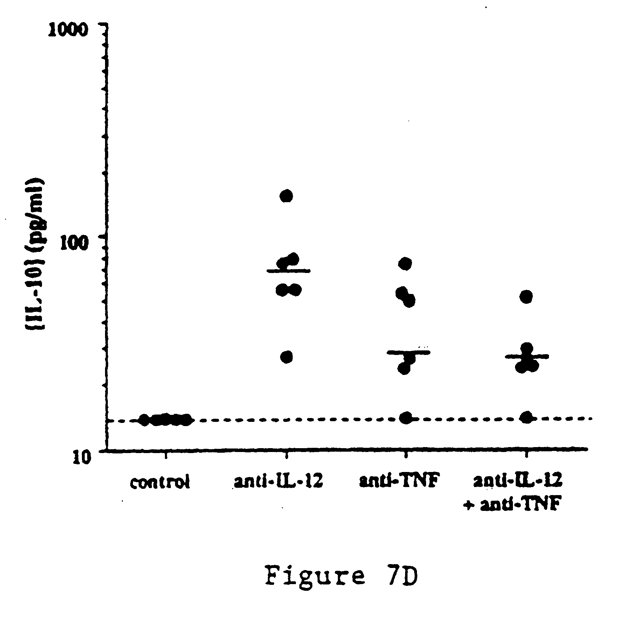

Figures 7A-7D are graphs showing in vitro IL-12 production (Figure 7A), IFNγ production (Figure 7E), TNF production (Figure 7C), and IL-10 production (Figure 7D) by synovial membrane cells from male DBA/1 mice with induced arthritis after treatment with anti-TNF antibody, anti-IL-12 antibody, or a combination of anti-TNF antibody and anti-IL-12 antibody. Median values are shown by a horizontal bar; dotted lines indicate detection levels.

### DETAILED DESCRIPTION OF THE INVENTION

The present invention relates to the unexpected and surprising discovery that co-administration of a TNF antagonist and an IL-12 antagonist in treating a TNF-mediated disease produces a significantly improved response compared to that obtained with administration of the TNF antagonist alone or that obtained with administration of the IL-12 antagonist alone. As a result of Applicants' invention, a method is provided herein for treating and/or preventing a TNF-mediated disease in an individual, comprising co-administering a tumor necrosis factor antagonist and an IL-12 antagonist to the individual in therapeutically effective amounts. The TNF antagonist and IL-12 antagonist can be administered simultaneously or sequentially. The anti-TNF antibody and anti-IL-12 antibody can each be administered in single or multiple doses. Multiple IL-12 antagonists and multiple TNF antagonists can be co-administered. Other therapeutic regimens and agents can be used in combination with the therapeutic co-administration of TNF antagonists and IL-12 antagonists.

Inflammatory mediators other than the described antagonists can be used instead of or in addition to these antagonists. As used herein, the term "inflammatory mediator" refers to an agent which decreases, blocks, inhibits, abrogates or interferes with pro-inflammatory mediator activity. Representative inflammatory mediators that can be used in the present invention include agents which decrease, block, inhibit, abrogate or interfere with IL-1 activity, synthesis, or receptor signalling, such as anti-IL-1 antibody, soluble IL-1R, IL-1 receptor antagonist, or other appropriate peptides and small molecules; agents which decrease, block, inhibit, abrogate or interfere with IL-6 activity, synthesis, or receptor signalling, such as anti-IL-6 antibody, anti-gp130, or other appropriate peptides and small molecules; modalities which decrease, block, inhibit, abrogate or interfere with the activity, synthesis, or receptor signalling of other pro-inflammatory mediators, such as GM-CSF and members of the chemokine IL-8 family; and cytokines with anti-inflammatory properties, such as IL-4, IL-10, IL-13, and TGFβ. In addition, other anti-inflammatory agents, such as the anti-rheumatic agent methotrexate, can be administered in conjunction with the IL-12 antagonist and/or the TNF antagonist. Inflammatory mediators and anti-inflammatory agents are also described in U.S. Patent No 6270766.

The present invention further relates to use in a method for treating and/or preventing recurrence of a TNF-mediated disease in an individual comprising co-administering a TNF antagonist and an IL-12 antagonist to the individual in therapeutically effective amounts.

As used herein, a "TNFα-mediated disease" refers to a TNFα related pathology or disease. TNFα related pathologies or diseases include, but are not limited to, the following:
(A) inflammatory diseases, including, but not limited to, acute and chronic immune and autoimmune pathologies, such as, but not limited to, rheumatoid arthritis (RA), juvenile chronic arthritis (JCA), spondyloarthropathy, thyroiditis, graft versus host disease (GVHD), scleroderma, diabetes mellitus, Graves' disease, allergy; acute or chronic immune disease associated with an allogenic transplantation, such as, but not limited to, renal transplantation, cardiac transplantation, bone marrow transplantation, liver transplantation, pancreatic transplantation, small intestine transplantation, lung transplantation and skin transplantation; chronic inflammatory pathologies, such as, but not limited to, sarcoidasis, chronic inflammatory bowel disease, ulcerative colitis, and Crohn's pathology or disease; vascular inflammatory pathologies, such as, but not limited to, disseminated intravascular coagulation, atherosclerosis, Kawasaki's pathology and vasculitis syndromes, such as, but not limited to, polyarteritis nodosa, Wegener's granulomatosis, Henoch-Schonlein purpura, giant cell arthritis and microscopic vasculitis of the kidneys; chronic active hepatitis; Sjögren's syndrome; spondyloarthropathies, such as ankylosing spondylitis, psoriatic arthritis and spondylitis, enteropathic arthritis and spondylitis, reactive arthritis and arthritis associated with inflammatory bowel disease; and uveitis;
(B) infections, including, but not limited to, sepsis syndrome, cachexia, circulatory collapse and shock resulting from acute or chronic bacterial infection, acute and chronic parasitic and/or infectious diseases, bacterial, viral or fungal, such as a human immunodeficiency virus (HIV), acquired immunodeficiency syndrome (AIDS) (including symptoms of cachexia, autoimmune disorders, AIDS dementia complex and infections);
(C) neurodegenerative diseases, including, but not limited to, demyelinating diseases, such as multiple sclerosis and acute transverse myelitis; myasthenia gravis; extrapyramidal and cerebellar disorders, such as lesions of the corcicospinal system; disorders of the basal ganglia or cerebellar disorders; hyperkinetic movement disorders, such as Huntington's chorea and senile chorea; drug-induced movement disorders, such as those induced by drugs which block central nervous system (CNS) dopamine receptors; hypokinetic movement disorders, such as Parkinson's disease; progressive supranuclear palsy; cerebellar and spinocerebellar disorders, such as astructural lesions of the cerebellum; spinocerebellar degenerations (spinal ataxia, Friedreich's ataxia, cerebellar cortical degenerations, multiple systems degenerations (Mencel, Dejerine-Thomas, Shi-Drager, and MachadoJoseph)); and systemic disorders (Refsum's disease, abetalipoproteinemia, ataxia, telangiectasia, and mitochondrial multisystem disorder); disorders of the motor unit, such as neurogenic muscular atrophies (anterior horn cell degeneration, such as amyotrophic lateral sclerosis, infantile spinal muscular atrophy and juvenile spinal muscular atrophy); Alzheimer's disease; Down's syndrome in middle age; diffuse Lewy body disease; senile dementia of Lewy body type; Wernicke-Korsakoff syndrome; chronic alcoholism; primary biliary cirrhosis; cryptogenic fibrosing alveolitis and other fibrotic lung diseases; hemolytic anemia; Creutzfeldt-Jakob disease; subacute sclerosing panencephalitis, Hallerrorden-Spatz disease; and dementia pugilistica, or any subset thereof;
(D) malignant pathologies involving TNF-secreting tumors or other malignancies involving TNF, such as, but not limited to, leukemias (acute, chronic myelocytic, chronic lymphocytic and/or myelodyspastic syndrome); lymphomas (Hodgkin's and non-Hodgkin's lymphomas, such as malignant lymphomas (Burkitt's lymphoma or Mycosis fungoides));
(E) cachectic syndromes and other pathologies and diseases involving excess TNFα, such as, but not limited to, cachexia of cancer, parasitic disease and heart failure; and
(F) alcohol-induced hepatitis and other forms of chronic hepatitis.

See, e.g., Berkow *et al*., Eds., *The Merck Manual*, 16th edition, chapter 11, pp. 1380-1529, Merck and Co., Rahway, New Jersey, 1992.

The terms "recurrence", "flare-up" or "relapse" are defined to encompass the reappearance of one or more symptoms of the disease state. For example, in the case of rheumatoid arthritis, a recurrence can include the experience of one or more of swollen joints, morning stiffness or joint tenderness.

In one embodiment, the invention relates to use in a method of treating and/or preventing rheumatoid arthritis in an individual comprising co-administering a TNF antagonist and an IL-12 antagonist to the individual in therapeutically effective amounts.

In a second embodiment, the invention relates to use in a method for treating and/or preventing Crohn's disease in an individual comprising co-administering a TNF antagonist and an IL-12 antagonist to the individual in therapeutically effective amounts.

In a third embodiment, the invention relates to use in a method for treating and/or preventing an acute or chronic immune disease associated with an allogenic transplantation in an individual comprising co-administering a TNF antagonist and an IL-12 antagonist to the individual in therapeutically effective amounts. As used herein, a "transplantation" includes organ, tissue or cell transplantation, such as renal transplantation, cardiac transplantation, bone marrow transplantation, liver transplantation, pancreatic transplantation, small intestine transplantation, skin transplantation and lung transplantation.

The benefits of combination therapy with TNF antagonists and IL-12 antagonists are significantly improved clinical response. A rapid and sustained reduction in the clinical signs and symptoms of the disease is possible. In addition, lower dosages can be used to provide the same reduction of the immune and inflammatory response, thus increasing the therapeutic window between a therapeutic and a toxic effect. Lower doses also result in lower financial costs to the patient, and potentially fewer side effects. For example, immune and/or allergic responses to TNF antagonists can be reduced, thus enhancing safety and therapeutic efficacy.

In a further embodiment, the invention relates to compositions comprising a TNF antagonist and an IL-12 antagonist. The compositions of the present invention are useful for treating a subject having a pathology or condition associated with abnormal levels of a substance reactive with a TNF antagonist, in particular TNP in excess of, or less than, levels present in a normal healthy subject, where such excess or diminished levels occur in a systemic, localized or particular tissue type or location in the body. Such tissue types can include, but are not limited to, blood, lymph, central nervous system (CNS), liver, kidney, spleen, heart muscle or blood vessels, brain or spinal cord white matter or grey matter, cartilage, ligaments, tendons, lung, pancreas, ovary, testes, prostate. Increased or decreased TNFα concentrations relative to normal levels can also be localized to specific regions or cells in the body, such as joints, nerve blood vessel junctions, bones, specific tendons or ligaments, or sites of infection, such as bacterial or viral infections.

### Tumor Necrosis Factor Antagonists

As used herein, a "tumor necrosis factor antagonist" decreases, blocks, inhibits, abrogates or interferes with TNFα activity in vivo. For example, a suitable TNF antagonist can bind TNFα and includes anti-TNF antibodies, antigen-binding fragments thereof, and receptor molecules and derivatives which bind specifically to TNFα. A suitable TNF antagonist can also prevent or inhibit TNF synthesis and/or TNF release and includes compounds such as thalidomide, tenidap, and phosphodiesterase inhibitors, such as, but not limited to, pentoxifylline and rolipram. A suitable TNF antagonist that can prevent or inhibit TNF synthesis and/or TNF release also includes A2b adenosine receptor enhancers and A2b adenosine receptor agonists (e.g., 5'-(N-cyclopropyl)-carboxamidoadenosine, 5'-N-ethylcarboxamidoadenosine, cyclohexyladenosine and R-N⁶-phenyl-2-propyladenosine). See, for example, Jacobson (GB 2 289 218 A). A suitable TNF antagonist can also prevent or inhibit TNF receptor signalling and includes mitogen activated protein (MAP) kinase inhibitors (e.g.., SB 203580; Lee and Young, *J. Leukocyte Biol. 59*:152-157 (1996). Other suitable TNF antagonists include agents which decrease, block, inhibit, abrogate or interfere with membrane TNF cleavage, such as, but not limited to, metalloproteinase inhibitors; agents which decrease, block, inhibit, abrogate or interfere with TNF activity, such as, but not limited to, angiotensin converting enzyme (ACE) inhibitors, such as captopril, enalapril and lisinopril; and agents which decrease, block, inhibit, abrogate or interfere with TNF production and/or synthesis, such as, but not limited to MAP kinase inhibitors. TNF antagonists are also described in CA-A-2261630 (published 12-02-1998),

International Publication No. WO 95/09652 (published April 13, 1995), U.S. Patent No 5741488 , International Publication No. WO 94/08619 (published April 28, 1994),

### Anti-TNF Antibodies

As used herein, an "anti-tumor necrosis factor antibody decreases, blocks, inhibits, abrogates or interferes with TNFα activity *in vivo*. In a preferred embodiment, the antibody specifically binds the antigen. The antibody can be polyclonal or monoclonal, and the term antibody is intended to encompass both polyclonal and monoclonal antibodies. The terms polyclonal and monoclonal refer to the degree of homogeneity of an antibody preparation, and are not intended to be limited to particular methods of production.

Suitable antibodies are available, or can be raised against an appropriate immunogen, such as isolated and/or recombinant antigen or portion thereof (including synthetic molecules, such as synthetic peptides) or against a host cell which expresses recombinant antigen. In addition, cells expressing recombinant antigen, such as transfected cells, can be used as immunogens or in a screen for antibody which binds receptor (see e.g., Chuntharapai et *al., J. Immunol., 152*: 1783-1789 (1994); and Chuntharapai *et al*., U.S. Patent No. 5,440,021).

Preparation of immunizing antigen, and polyclonal and monoclonal antibody production can be performed using any suitable technique. A variety of methods have been described (see e.g., Kohler *et al., Nature, 256:* 495-497 (1975) and *Eur. J. Immunol., 6:* 511-519 (1976); Milstein *et al., Nature, 266: 550-552 (1977);* Koprowski *et al*., U.S. Patent No. 4,172,124; Harlow, E. and D. Lane, 1988, *Antibodies: A Laboratory Manual*, (Cold Spring Harbor Laboratory: Cold Spring Harbor, NY); and *Current Protocols In Molecular Biology*, Vol. 2 (Supplement 27, Summer '94), Ausubel *et al*., Eds., (John Wiley & Sons: New York, NY), Chapter 11, (1991)). Generally, a hybridoma can be produced by fusing a suitable immortal cell line (e.g., a myeloma cell line such as SP2/0) with antibody producing cells. The antibody producing cell, preferably those of the spleen or lymph nodes, can be obtained from animals immunized with the antigen of interest. The fused cells (hybridomas) can be isolated using selective culture conditions, and cloned by limiting dilution. Cells which produce antibodies with the desired specificity can be selected by a suitable assay (e.g., ELISA).

Other suitable methods of producing or isolating antibodies of the requisite specificity, including human antibodies, can be used, including, for example, methods by which a recombinant antibody or portion thereof are selected from a library, such as, for example, by phage display technology (see, e.g., Winters *et al., Annu. Rev*. *Immunol., 12*:433-455 (1994); Hoogenboom *et al*., WO 93/06213; Hoogenboom *et al*., U.S. Patent No. 5,565,332; WO 94/13804, published June 23, 1994; Krebber *et al*., U.S. Patent No. 5,514,548; and Dower *et al*., U.S. Patent No. 5,427,908), or which rely upon immunization of transgenic animals (e.g., mice) capable of producing a full repertoire of human antibodies (see e.g., Jakobovits *et al*., *Proc. Natl. Acad. Sci. USA, 90*: 2551-2555 (1993); Jakobovits *et al., Nature, 362*: 255-258 (1993); Kucherlapati *et al*., European Patent No. EP 0 463 151 B1; Lonberg *et al*., U.S. Patent No. 5,569,825; Lonberg *et al*., U.S. Patent No. 5,545,806; and Surani *et al*., U.S. Patent No. 5,545,807).

Single chain antibodies, and chimeric, humanized or primatized (CDR-grafted antibodies, with or without framework changes), or veneered antibodies, as well as chimeric, CDR-grafted or veneered single chain antibodies, comprising portions derived from different species, and the like are also encompassed by the present invention and the term "antibody". The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly *et al*., U.S. Patent No. 4,816,567; Cabilly *et al*., European Patent No. 0,125,023 B1; Boss *et al*., U.S. Patent No. 4,816,397; Boss *et al*., European Patent No. 0,120,694 B1; Neuberger, M.S. *et al*., WO 86/01533; Neuberger, M.S. *et al*., European Patent No. 0,194,276 B1; Winter, U.S. Patent No. 5,225,539; Winter, European Patent No. 0,239,400 B1; Queen *et al*., U.S. Patent No. 5,585,089; Queen *et al*., European Patent No. 0,451,216 B1; Adair *et al*., WO 91/09967, published 11 July 1991; Adair *et al*., European Patent No. 0,460,167 B1; and Padlan, E.A. *et al*., European Patent No. 0,519,596 A1. See also, Newman, R. *et al*., *BioTechnology, 10:* 1455-1460 (1992), regarding primatized antibody, and Huston *et al*., U.S. Patent No. 5,091,513; Huston *et al*., U.S. Patent No. 5,132,405; Ladner *et al*., U.S. Patent No. 4,946,778 and Bird, R.E. *et al., Science*, 242: 423-426 (1988)) regarding single chain antibodies.

In addition, antigen binding fragments of antibodies, including fragments of chimeric, humanized, primatized, veneered or single chain antibodies and the like, can also be produced. For example, antigen binding fragments include, but are not limited to, fragments such as Fv, Fab, Fab' and F(ab')₂ fragments. Antigen binding fragments can be produced by enzymatic cleavage or by recombinant techniques, for example. For instance, papain or pepsin cleavage can generate Fab or F(ab')₂ fragments, respectively. Antibodies can also be produced in a variety of truncated forms using antibody genes in which one or more stop codons has been introduced upstream of the natural stop site. For example, a chimeric gene encoding a F(ab')₂ heavy chain portion can be designed to include DNA sequences encoding the CH₂ domain and hinge region of the heavy chain.

Anti-TNF antibodies useful in the methods and compositions of the present invention are characterized by high affinity binding to TNFα and low toxicity (including human anti-murine antibody (HAMA) and/or human anti-chimeric antibody (HACA) response). In particular, an antibody where the individual components, such as the variable region, constant region and framework, individually and/or collectively possess low immunogenicity is useful in the present invention. The antibodies which can be used in the invention are characterized by their ability to treat patients for extended periods with good to excellent alleviation of symptoms and low toxicity. Low immunogenicity and/or high affinity, as well as other undefined properties, may contribute to the therapeutic results achieved. "Low immunogenicity" is defined herein as raising significant HACA or HAMA responses in less than about 75%, or preferably less than about 50% of the patients treated and/or raising low titres in the patient treated (less than about 300, preferably less than about 100 measured with a double antigen enzyme immunoassay) (Elliott *et al., Lancet 344*:1125-1127 (1994)).

In a particular embodiment, the anti-TNFα antibody is chimeric monoclonal antibody cA2 (or an antigen binding f ragment thereof) or murine monoclonal antibody A2 (or an antigen binding fragment thereof), or has an epitopic specificity similar to that of chimeric antibody cA2, murine monoclonal antibody A2, or antigen binding fragments thereof, including antibodies or antigen binding fragments reactive with the same or a functionally equivalent epitope on human TNFα as that bound by chimeric antibody cA2 or murine monoclonal antibody A2, or antigen binding fragments thereof. Antibodies with an epitopic specificity similar to that of chimeric antibody cA2 or murine monoclonal antibody A2 include antibodies which can compete with chimeric antibody cA2 or murine monoclonal antibody A2 (or antigen binding fragments thereof) for binding to human TNFα. such antibodies or fragments can be obtained as described above. Chimeric antibody cA2, murine monoclonal antibody A2 and methods of obtaining these antibodies are also described in U.S. Patent No 5698195, US Patent No 5656272,

Le, J. *et al*., International Publication No. WO 92/16553 (published October 1, 1992), Knight, D.M. *et al., Mol. Immunol., 30*:1443-1453 (1993), and Siegel, S.A. *et al., Cytokine, 7*(1):15-25 (1995).

Chimeric antibody cA2 consists of the antigen binding variable region of the high-affinity neutralizing mouse anti-human TNFα IgG1 antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular embodiment, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the affinity constant of chimeric antibody cA2 was calculated to be 1.04x10¹⁰M⁻¹. Preferred methods for determining monoclonal antibody specificity and affinity by competitive inhibition can be found in Harlow, *et al., Antibodies: A Laboratory Manual*, Cold Spring Harbor Laboratory Press, Cold Spring Harbor, New York, 1988; Colligan *et al., eds., Current Protocols in Immunology*, Greene Publishing Assoc. and Wiley Interacience, New York, (1992, 1993); Kozbor *et al., Immunol. Today, 4*:72-79 (1983); Ausubel *et al., eds. Current Protocols in Molecular Biology*, Wiley Interscience, New York (1987, 1992, 1993); and Muller, *Meth. Enzymol., 92*:589-601 (1983).

In a particular embodiment, murine monoclonal antibody A2 is produced by a cell line designated c134A. Chimeric antibody cA2 is produced by a cell line designated c168A.

Additional examples of monoclonal anti-TNF antibodies that can be used in the present invention are described in the art (see, e.g., U.S. Patent No. 5,231,024; Möller, A. *et al., Cytokine 2*(3):162-169 (1990); U.S. Application No. 07/943,852 (filed September 11, 1992); Rathjen *et al*., International Publication No. WO 91/02078 (published February 21, 1991); Rubin *et al*., EPO Patent Publication No. 0 218 868 (published April 22, 1987); Yone *et al*., EPO Patent Publication No. 0 288 088 (October 26, 1988); Liang, *et al., Biochem. Biophys. Res. Comm. 137*:847-854 (1986); Meager, *et al., Hybridoma 6*:305-311 (1987); Fendly *et al*., *Hybridoma 6*:359-369 (1987); Bringman, *et al., Hybridoma 6*:489-507 (1987); and Hirai, *et al., J. Immunol. Meth.* 96:57-62 (1987).

As used herein, the term "antigen binding region" refers to that portion of an antibody molecule which contains the amino acid residues that interact with an antigen and confer on the antibody its specificity and affinity for the antigen. The antigen binding region includes the "framework" amino acid residues necessary to maintain the proper conformation of the antigen-binding residues.

The term antigen refers to a molecule or a portion of a molecule capable of being bound by an antibody which is additionally capable of inducing an animal to produce antibody capable of selectively binding to an epitope of that antigen. An antigen can have one or more than one epitope.

The term epitope is meant to refer to that portion of the antigen capable of being recognized by and bound by an antibody at one or more of the antibody's antigen binding region. Epitopes usually consist of chemically active surface groupings of molecules such as amino acids or sugar side chains and have specific three dimensional structural characteristics as well as specific charge characteristics. By "inhibiting and/or neutralizing epitope" is intended an epitope, which, when bound by an antibody, results in loss of biological activity of the molecule containing the epitope, *in vivo* or *in vitro*, more preferably in *vivo*, including binding of TNFα to a TNFα receptor.

### TNF Receptor Molecules

TNF receptor molecules useful in the methods and compositions of the present invention are those that bind TNFα with high affinity (see, e.g., Feldmann *et al*., International Publication No. WO 92/07076 (published April 30, 1992); Schall *et al., Cell 61*:361-370 (1990); and Loetscher *et al., Cell 61*:351-359 (1990)) and possess low immunogenicity. In particular, the 55 kDa (p55 TNF-R) and the 75 kDa (p75 TNF-R) TNF cell surface receptors are useful in the present invention. Truncated forms of these receptors, comprising the extracellular domains (ECD) of the receptors or functional portions thereof (see, e.g., Corcoran *et al., Eur. J. Biochem. 223*:831-840 (1994)), are also useful in the present invention. Truncated forms of the TNF receptors, comprising the ECD, have been detected in urine and serum as 30 kDa and 40 kDa TNFα inhibitory binding proteins (Engelmann, H. *et al*., *J. Biol. Chem*. *265*:1531-1536 (1990)). TNF receptor multimeric molecules and TNF immunoreceptor fusion molecules, and derivatives and fragments or portions thereof, are additional examples of TNF receptor molecules which are useful in the methods and compositions of the present invention. The TNF receptor molecules which can be used in the invention are characterized by their ability to treat patients for extended periods with good to excellent alleviation of symptoms and low toxicity. Low immunogenicity and/or high affinity, as well as other undefined properties, may contribute to the therapeutic results achieved.

TNF receptor multimeric molecules useful in the present invention comprise all or a functional portion of the ECD of two or more TNF receptors linked via one or more polypeptide linkers or other nonpeptide linkers, such as polyethylene glycol (PEG). The multimeric molecules can further comprise a signal peptide of a secreted protein to direct expression of the multimeric molecule.

TNF immunoreceptor fusion molecules useful in the methods and compositions of the present invention comprise at least one portion of one or more immunoglobulin molecules and all or a functional portion of one or more TNF receptors. These immunoreceptor fusion molecules can be assembled as monomers, or hetero- or homo-multimers. The immunoreceptor fusion molecules can also be monovalent or multivalent. An example of such a TNF immunoreceptor fusion molecule is TNF receptor/IgG fusion protein.

TNF immunoreceptor fusion molecules and methods for their production have been described in the art (Lesslauer *et al., Eur. J. Immunol. 21*:2883-2886 (1991); Ashkenazi *et al., Proc. Natl. Acad. Sci. USA 88*:10535-10539 (1991); Peppel *et al., J. Exp. Med. 174*:1483-1489 (1991); Kolls *et al., Proc. Natl. Acad. Sci. USA 91*:215-219 (1994); Butler *et al., Cytokine 6*(6):616-623 (1994); Baker *et al., Bur. J. Immunol. 24*:2040-2048 (1994); Beutler *et al*., U.S. Patent No. 5,447,851. Methods for producing immunoreceptor fusion molecules can also be found in Capon *et al*., U.S. Patent No. 5,116,964; Capon *et al*., U.S. Patent No. 5,225,538; and Capon *et al., Nature 337*:525-531 (1989).

A functional equivalent, derivative, fragment or region of TNF receptor molecule refers to the portion of the TNF receptor molecule, or the portion of the TNF receptor molecule sequence which encodes TNF receptor molecule, that is of sufficient size and sequences to functionally resemble TNF receptor molecules that can be used in the present invention (e.g., bind TNFα with high affinity and possess low immunogenicity). A functional equivalent of TNF receptor molecule also includes modified TNF receptor molecules that functionally resemble TNF receptor molecules that can be used in the present invention (e.g., bind TNFα with high affinity and possess low immunogenicity). For example, a functional equivalent of TNF receptor molecule can contain a "SILENT" codon or one or more amino acid substitutions, deletions or additions (e.g., substitution of one acidic amino acid for another acidic amino acid; or substitution of one codon encoding the same or different hydrophobic amino acid for another codon encoding a hydrophobic amino acid). See Ausubel *et al., Current Protocols in Molecular Biology*, Greene Publishing Assoc. and Wiley-Interscience, New York (1989).

### IL-12 Antagonists

As used herein, an "IL-12 antagonist" decreases, blocks, inhibits, abrogates or interferes with IL-12 activity, synthesis or receptor signalling *in vivo*. IL-12 antagonists include anti-IL-12 antibodies, receptor molecules which bind specifically to IL-12, IL-12 receptor antagonists, IL-12 p40 homodimers, IL-12 p35 homodimers, and other appropriate peptides and small molecules. IL-12 p40 homodimers are described in the art (see, e.g., Ling *et al., J. Immunol. 154*(1):116-127 (1995); and Gillessen *et al., Eur. J. Immunol. 25*(1) :200-206 (1995). IL-12 antagonists also include agents which decrease, inhibit, block, abrogate or interfere with IL-12 production, such as compounds (e.g. drugs and other agents, including antibodies) which inhibit, block, abrogate or interfere with CD40 or its ligands; adrenergic agonists, such as, but not limited to, salbutamol; and cytokines, such as IL-4, IL-10, IL-13 and TGFβ. IL-12 antagonists also include agents (e.g., drugs and other agents) which decrease, abrogate, block, inhibit or interfere with IL-12 receptor signalling.

### Anti-IL-12 Antibodies

As used herein, an anti-IL-12 antibody (or an antigen binding fragment thereof) decreases, blocks, inhibits, abrogates or interferes with IL-12 activity *in vivo*. Antibodies and antigen binding fragments are as described above. For example, the antibody can be polyclonal or monoclonal. As above, the antibody can be a single chain chimeric, humanized, primatized or veneered antibody. Such antibodies or fragments can be obtained as described above. Advantageously, anti-IL-12 antibodies (and antigen binding fragments thereof) are characterized by high affinity binding to IL-12 and low toxicity (including HAMA and/or HACA response). An antibody where the individual components, such as the variable region, constant region and framework, individually and/or collectively possess low immunogenicity is particularly useful. In a particular embodiment, anti-IL-12 antibodies (and antigen binding fragments thereof) are characterized by their ability to treat patients for extended periods with good to excellent alleviation of symptoms and low toxicity. Low immunogenicity and/or high affinity, as well as other undefined properties, may contribute to the therapeutic results achieved.

Examples of anti-IL-12 antibodies that can be used in the present invention are described in the art (see, e.g., Wilkinson *et al., J. Immunol. Methods 189*:15-24 (1996); Tripp *et al., J. Immunol. 152*:1883-1887 (1994); Trinchieri *et al., Blood 84*:4008-4027 (1994); Wysocka *et al., Eur. J*. *Immunol. 25*:672-676 (1995); Neurath *et al., J. Exp. Med. 182*:1281-1290 (1995); Chizzonite *et al., J. Immunol*. *147*(5):1548-1556 (1991); Ozmen *et al., J. Exp. Med*. *180*:907-915 (1994); D'Andrea *et al., J. Exp. Med. 176*:1387-1398 (1992); Ozmen *et al., J. Exp. Med. 180*:907-915 (1994); Riemann *et al., J. Immunol*. *156*(5):1799-1803 (1996); Gazzinelli *et al., J. Immunol*. 153(6):2533-2543 (1994); Jones, *Scand. J. Immunol*. *43*(1):64-72 (1996); Sypek *et al., J. Exp. Med*. *177*:1797-1802 (1993); and Valiante *et al., Cell Immunol*. *145*(1):187-198 (1992).

### IL-12 Receptor Molecules

IL-12 receptor molecules useful in the present invention bind specifically to IL-12 and possess low immunogenicity. Preferably, the IL-12 receptor molecule is characterized by high affinity binding to IL-12. IL-12 receptor molecules include IL-12 receptors, IL-12 receptor multimeric molecules and IL-12 immunoreceptor fusion molecules, and derivatives and fragments or portions thereof. An example of an IL-12 receptor molecule is described by Chua *et al*. (U.S. Application No. 5,536,657; and Chua *et al., J. Immunol. 153*:128-136 (1994). The IL-12 receptor molecules which can be used in the invention are characterized by their ability to treat patients for extended periods with good to excellent alleviation of symptoms and low toxicity. Low immunogenicity and/or high affinity, as well as other undefined properties, may contribute to the therapeutic results achieved.

IL-12 receptor multimeric molecules can comprise all or a functional portion of two or more IL-12 receptors linked via one or more linkers. IL-12 receptor multimeric molecules can further comprise a signal peptide of a secreted protein to direct expression of the multimeric molecule.

IL-12 immunoreceptor fusion molecules can comprise at least one portion of one or more immunoglobulin molecules and all or a functional portion of one or more IL-12 receptors. IL-12 immunoreceptor fusion molecules can be assembled as monomers, or hetero- or homo-multimers. IL-12 immunoreceptor fusion molecules can also be monovalent or multivalent.

A functional equivalent, derivative, fragment or region of IL-12 receptor molecule refers to the portion of the IL-12 receptor molecule, or the portion of the IL-12 receptor molecule sequence which encodes IL-12 receptor molecule, that is of sufficient size and sequences to functionally resemble IL-12 receptor molecules that can be used in the present invention (e.g., bind specifically to IL-12 and possess low immunogenicity). A functional equivalent of IL-12 receptor molecule also includes modified IL-12 receptor molecules that functionally resemble IL-12 receptor molecules that can be used in the present invention (e.g., bind specifically to IL-12 and possess low immunogenicity). For example, a functional equivalent of IL-12 receptor molecule can contain a "SILENT" codon or one or more amino acid substitutions, deletions or additions. For example, a functional equivalent of IL-12 receptor molecule can contain a substitution of one acidic amino acid for another acidic amino acid, or a substitution of one codon encoding the same or different hydrophobic amino acid for another codon encoding a hydrophobic amino acid. See Ausubel *et al*., *Current Protocols in Molecular Biology*, Greene Publishing Assoc. and Wiley-Interscience, New York (1989).

Techniques described herein for producing TNF receptor molecules can be employed in producing IL-12 receptor molecules that can be used in the present invention.

### Administration

TNF antagonists, IL-12 antagonists, and the compositions of the present invention can be administered to an individual in a variety of ways. The routes of administration include intradermal, transdermal (e.g., in slow release polymers), intramuscular, intraperitoneal, intravenous (including infusion and/or bolus injection), subcutaneous, oral, topical, epidural, buccal, rectal, vaginal and intranasal routes. Other suitable routes of administration can also be used, for example, to achieve absorption through epithelial or mucocutaneous linings. TNF antagonists, IL-12 antagonists, and compositions of the present invention can also be administered by gene therapy wherein a DNA molecule encoding a particular therapeutic protein or peptide is administered to the patient, e.g., via a vector, which causes the particular protein or peptide to be expressed and secreted at therapeutic levels in vivo. In addition, TNF antagonists, IL-12 antagonists, and compositions of the present invention can be administered together with other components of biologically active agents, such as pharmaceutically acceptable surfactants (e.g., glycerides), excipients (e.g., lactose), carriers, diluents and vehicles. If desired, certain sweetening, flavoring and/or coloring agents can also be added.

TNF antagonists, IL-12 antagonists and compositions of the present invention can be administered prophylactically or therapeutically to an individual. TNF antagonists can be administered prior to, simultaneously with (in the same or different compositions) or sequentially with the administration of an IL-12 antagonist.

For parenteral (e.g., intravenous, subcutaneous, intramuscular) administration, TNF antagonists, IL-12 antagonists and compositions of the present invention can be formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils can also be used. The vehicle or lyophilized powder can contain additives that maintain isotonicity (e.g., sodium chloride, mannitol) and chemical stability (e.g., buffers and preservatives). The formulation is sterilized by commonly used techniques.

Suitable pharmaceutical carriers are described in Remington's Pharmaceutical Sciences.

For example, a parenteral composition suitable for administration by injection is prepared by dissolving 1.5% by weight of active ingredient in 0.9% sodium chloride solution.

TNF antagonists and IL-12 antagonists are co-administered simultaneously or sequentially in therapeutically effective amounts; compositions of the present invention are administered in a therapeutically effective amount. As used herein, a "therapeutically effective amount" is such that co-administration of TNF antagonist and IL-12 antagonist, or administration of a composition of the present invention, results in inhibition of the biological activity of TNFα relative to the biological activity of TNFα when therapeutically effective amounts of TNF antagonist and IL-12 antagonist are not co-administered, or relative to the biological activity of TNFα when a therapeutically effective amount of the composition is not administered. A therapeutically effective amount is that amount of TNF antagonist and IL-12 antagonist necessary to significantly reduce or eliminate symptoms associated with a particular TNFα-mediated disease. As used herein, a therapeutically effective amount is not an amount such that administration of the TNF antagonist alone, or administration of the IL-12 antagonist alone, must necessarily result in inhibition of the biological activity of TNFα or in immunosuppressive activity.

Once a therapeutically effective amount has been administered, a maintenance amount of TNF antagonist alone, of IL-12 antagonist alone, or of a combination of TNF antagonist and IL-12 antagonist can be administered to the individual. A maintenance amount is the amount of TNF antagonist, IL-12 antagonist, or combination of TNF antagonist and IL-12 antagonist necessary to maintain the reduction or elimination of symptoms achieved by the therapeutically effective dose. The maintenance amount can be administered in the form of a single dose, or a series or doses separated by intervals of days or weeks.

The dosage administered to an individual will vary depending upon a variety of factors, including the pharmacodynamic characteristics of the particular antagonists, and its mode and route of administration; size, age, health, sex, body weight and diet of the recipient; nature and extent of symptoms of the disease being treated, kind of concurrent treatment, frequency of treatment, and the effect desired. In vitro and in vivo methods of determining the inhibition of TNF in an individual are well known to those of skill in the art. Such in vitro assays can include a TNF cytotoxicity assay (e.g., the WEHI assay or a radioimmunoassay, ELISA). In vivo methods can include rodent lethality assays, primate pathology model systems (see, e.g., Mathison *et al., J. Clin. Invest. 81*: 1925-1937 (1988); Beutler *et al., Science 229*: 869-871 (1985); Tracey *et al., Nature 330:* 662-664 (1987); Shimamoto *et al., Immunol. Lett. 17*: 311-318 (1988); Silva *et al., J. Infect. Dis. 162*: 421-427 (1990); Opal *et al., J. Infect. Dis. 161*: 1148-1152 (1990); and Hinshaw *et al., Circ. Shock 30*: 279-292 (1990)) and/or rodent models of arthritis (Williams *et al., Proc. Natl*. *Acad. Sci. USA 89*:9784-9788 (1992)). In patients with rheumatoid arthritis, TNFα blockade can be monitored by monitoring IL-6 and C-reactive protein levels (Elliott *et al., Arthritis Rheum. 36*:1681-1690 (1993)).

TNF antagonists and IL-12 antagonists can be co-administered in single or multiple doses depending upon factors such as nature and extent of symptoms, kind of concurrent treatment and the effect desired. Thus, other therapeutic regimens or agents (e.g., multiple drug regimens) can be used in combination with the therapeutic co-administration of TNF antagonists and IL-12 antagonists. Adjustment and manipulation of established dosage ranges are well within the ability of those skilled in the art.

Usually a daily dosage of active ingredient can be about 0.01 to 100 milligrams per kilogram of body weight. Ordinarily 1 to 40 milligrams per kilogram per day given in divided doses 1 to 6 times a day or in sustained release form is effective to obtain desired results. Second or subsequent administrations can be administered at a dosage which is the same, less than or greater than the initial or previous dose administered to the individual.

A second or subsequent administration is preferably during or immediately prior to relapse or a flare-up of the disease or symptoms of the disease. For example, the second and subsequent administrations can be given between about one day to 30 weeks from the previous administration. Two, three, four or more total administrations can be delivered to the individual, as needed.

Dosage forms (composition) suitable for internal administration generally contain from about 0.1 milligram to about 500 milligrams of active ingredient per unit. In these pharmaceutical compositions the active ingredient will ordinarily be present in an amount of about 0.5-95% by weight based on the total weight of the composition.

The present invention will now be illustrated by the following examples, which are not intended to be limiting in any way.

### EXAMPLE

### EXAMPLE Treatment of Induced Arthritis in a Murine Model Using Anti-TNF Antibody and Anti-IL-12 Antibody

The murine model of type II collagen induced arthritis (CIA) has similarities to rheumatoid arthritis (RA) in its marked MHC class II predisposition, as well as in histology, immunohistology, and erosions of cartilage and bone. Furthermore, there is a good correlation of its therapeutic response with that of human rheumatoid arthritis. For example, in both diseases anti-TNF antibody has beneficial effects (Williams, R.O. *et al., Proc. Natl*. *Acad. Sci. USA 89*:9784-9788 (1992); Elliott, M.J. *et al*., *Arthritis & Rheumatism 36*:1681-1690 (1993)). Thus, the animal model serves as a good approximation to human disease.

The model of rheumatoid arthritis used herein, i.e., the type II collagen induced arthritis in the DBA/1 mouse, is described by Williams, R.O. *et al. (Proc. Natl. Acad. Sci. USA 89*:9784-9788 (1992)). Type II collagen (CII) was purified from bovine hyaline cartilage by limited pepsin solubilization and salt fractionation as described by Miller (Biochemistry 11:4903-4909 (1972)).

### Anti-IL-12 Antibody

The anti-IL-12 p40 antibody (clone 17.8) is a rat anti-murine IgG2a monoclonal antibody provided by Giorgio Trinchieri at the Wistar Institute (Philadelphia, PA) (Trinchieri *et al., Blood 84*:4008-4027 (1994); Wysocka *et al., Eur.* J. *Immunol. 25*: 672-676 (1995); and Neurath *et al., J. Exp. Med. 182*:1281-1290 (1995).

### Anti-TNF Antibody

The anti-TNFα antibody cV1q was constructed by Centocor, Inc. (Malvern, PA). Hybridoma cells secreting the rat anti-murine TNFα antibody V1q were from Peter Krammer at the German Cancer Research Center, Heidelberg, Germany (Echtenacher *et al., J. Immunol. 145*:3762-3766 (1990)). Genes encoding the variable regions of the heavy and light chains of the anti-TNFα antibody V1q were cloned. The cloned heavy chain was inserted into four different gene expression vectors to encode cV1q heavy chain with either a human IgG1, human IgG3, murine IgG1 or murine IgG2a constant region. The V1q light chain gene was inserted into other expression vectors to encode either a human kappa or a murine kappa light chain constant region.

SP2/O myeloma cells were transfected with the different heavy and light chain gene constructs. Cell clones producing chimeric V1q (cV1q) were identified by assaying cell supernatant for human or murine IgG using standard ELISA assays. High-producing clones were subcloned to obtain homogenous cell lines. The murine IgG1 and IgG2a versions are referred to as C257A and C258A, respectively. cV1q was purified from cell supernatant by protein A chromatography.

cV1q was characterized by measuring its affinity for soluble murine TNFα, testing its ability to protect WEHI cells from murine TNFα cytotoxicity, examining its ability to neutralize or bind murine lymphotoxin, comparing the ability of the murine IgG1 and IgG2a versions to trigger complement-mediated lysis of cells expressing recombinant transmembrane murine TNFα, and examining the ability of the human IgG1 version to protect mice from lethal doses of LPS (endotoxin). The murine IgG1 version of cV1q was used in the following experimental procedure.

### Experimental Procedure

Male DBA/1 mice were immunized intradermally at the base of the tail at 8-12 weeks of age with 100 µg type II collagen emulsified in Freund's complete adjuvant (Difco Laboratories, Detroit, MI). From day 15 after immunization onwards, mice were examined daily for onset of disease using two clinical parameters: paw swelling and clinical score.

Paw-swelling was assessed by measuring the thickness of the affected hindpaws with 0-10 mm calipers.

Clinical score was assessed on the following scale: 0 = normal; 1 = slight swelling and erythema; 2 = pronounced edema; and 3 = joint rigidity. Each limb was graded, giving a maximum clinical score of 12 per mouse.

Day one of arthritis was considered to be the day that erythema and/or swelling in one or more limbs was first observed. Arthritis became clinically evident around 18 to 30 days (average 23 days) after immunization with type II collagen. After the onset of clinically evident arthritis, two groups of mice (Group 1 and Group 2; 9 mice per group) were subjected to treatment with one of the following therapies: 500 µg/ml anti-IL-12 p40 antibody (clone 17.8), injected intra-peritoneally once every other day (days 1, 3, 5, 7 and 9); 300 µg/ml anti-TNF antibody cV1q, injected intra-peritoneally once every other day (days 1, 3, 5, 7 and 9); 500 µg/ml anti-IL-12 p40 antibody (clone 17.8), injected intra-peritoneally once every other day (days 1, 3, 5, 7 and 9) in conjunction with 300 µg/ml anti-TNF antibody cV1q, injected intra-peritoneally once every other day (days 1, 3, 5, 7 and 9); or phosphate-buffered saline (PBS; control), injected intra-peritoneally once every other day.

Arthritis in the mice was monitored using paw-swelling and clinical score over a 10 day period, after which the mice were sacrificed and joints were processed for histology. The Mann-Whitney U test was applied to all clinical results and cell culture experiments to compare non-parametric data for statistical significance. The chi-square test was used to analyze histological data.

### Paw-Swelling

Treatment with a combination of anti-TNF antibody and anti-IL-12 antibody resulted in a highly significant and sustained reduction in paw-swelling over the treatment period. Results are shown in Figure 1B (Group 1) and Figure 2A (Group 2). p-values are given for the combination treated mice versus the PBS treated mice (Mann-Whitney U test).

### Clinical Score

Clinical score results are presented in Figure 1A (Group 1) and Figure 2B (Group 2) and confirm that treatment with a combination of anti-TNF antibody and anti-IL-12 antibody significantly ameliorated disease. That is, when anti-TNF antibody and anti-IL-12 antibody are administered together, there was a highly significant reduction in the severity of arthritis, p-values are given for the combination treated mice versus the PBS treated mice (Mann-Whitney U test).

### Histology

Hindpaws and knee joints were removed post mortem on the tenth day of arthritis, fixed in formalin and decalcified in 5% EDTA. Paws and knees were then embedded in paraffin, sectioned and stained with hematoxylin and eosin. Arthritic changes in the ankle, the metatarsophalangeal joints, the proximal interphalangeal and the distal interphalangeal joints were scored blindly as mild (= mild synovial hyperplasia); moderate (= pannus formation and erosions limited to the cartilage-pannus junction); or severe (= extended bone and cartilage erosions with loss of joint architecture).

Results are presented in Figures 3A and 3B. Blinded histological analysis of the joints in all the hind paws of the control mice (mice treated with PBS) showed that all were affected and 68% were destroyed, while the rest showed moderate or mild damage. Anti-TNF antibody treatment reduced the numbers of joints with severe erosions to 20%, but normal joints were not found in the paws of these mice. In spite of the failure to modify the clinical inflammatory features of CIA, anti-IL-12 antibody treatment was found to reduce the number of joints afflicted, with 18% being completely normal, but the level of damage in those joints that were affected was similar to that observed in the control mice. As indicated by the clinical results, administration of a combination of anti-TNF antibody and anti-IL-12 antibody was highly successful in preventing joint damage and significantly more joints in the hind paw were normal or mildly affected compared to the controls (p < 0.001) (Figure 3A).

Histological analysis of the knees revealed that treatment with a combination of anti-TNF antibody and anti-IL-12 antibody also successfully alleviated arthritis at this site (p = 0.05), with 45% of the mice displaying normal knee joints and a reduced number with moderate or severe damage, compared to the other treatment groups (Figure 3B).

Thus, significantly more joints in the hind paws were scored as "normal" or "mild" in the mice treated with a combination of anti-TNF antibody and anti-IL-12 antibody compared to the mice treated with PBS (Figure 3A; p < 0.01, chi-square test). Similar results were observed in the knee joints (Figure 3B; p = 0.05).

### Anti-Collagen IgG Levels

Mice were bled *post mortem*, i.e., after 10 or 20 days of arthritis. Serum levels of anti-CII antibodies (IgG1 and IgG2a subclasses), and of total IgG anti-CII antibodies, were measured by modification of an enzyme-linked immunosorbent assay (ELISA) as described in detail for the detection of human IgG (Williams *et al., Proc. Natl. Acad. Sci. USA 89*:9784-9788 (1992)). Briefly, microtitre plates were coated with native bovine type II collagen (2 µg/ml), blocked, then incubated with serially diluted test sera. Bound IgG was detected by incubation with alkaline phosphatase-conjugated sheep anti-mouse IgG1 or IgG2a (Binding Site, Birmingham, UK), or goat anti-mouse IgG (Jackson ImmunoResearch, Luton, UK), followed by substrate (dinitrophenyl phosphate). Plates were washed between steps with 0.01% Tween-PBS. Optical densities were read at 405 nm.

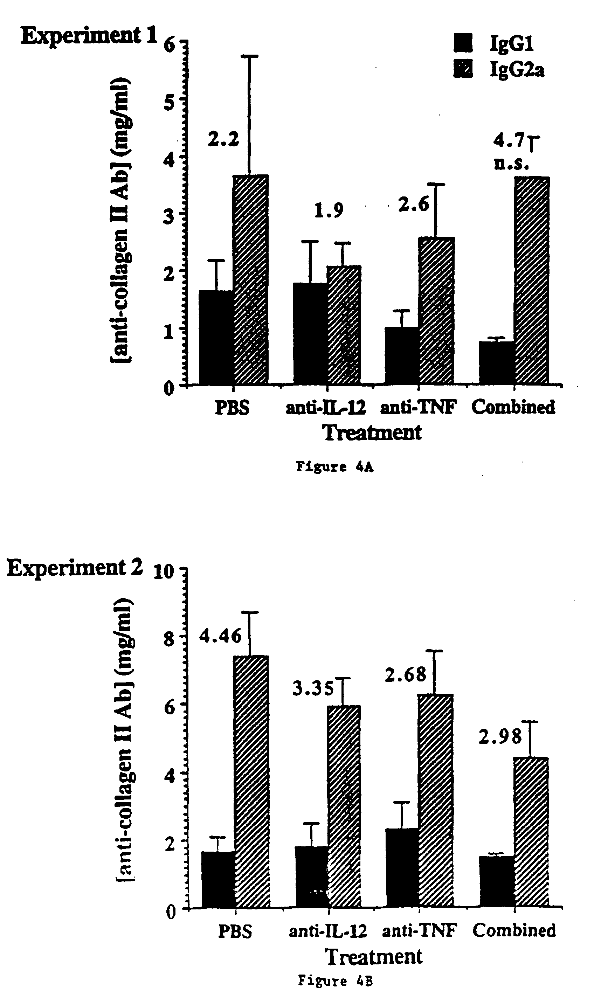

The serum levels of IgG1 and IgG2a anti-CII antibodies are shown in Figure 4A (Group 1), Figure 4B (Group 2) and Figure 4C (measured after 20 days of arthritis) for each treatment regimen. The ratio of serum IgG2a/IgG1 subclasses is given for each treatment regimen. Serum levels of IgG1 and IgG2a anti-CII antibodies, as well as the ratio of serum IgG2a/IgG1 subclasses, were not significantly altered within the 10 day treatment period by anti-TNF antibody alone, anti-IL-12 antibody alone or anti-TNF antibody plus anti-IL-12 antibody. However, measurements at day 20 of arthritis showed that the ratio of serum IgG2a/IgG1 subclasses was reduced from 9 to 4 in the mice treated by anti-TNF antibody alone and from 9 to 5 in the mice treated with anti-TNF antibody plus anti-IL-12 antibody (Figure 4C). The change in ratio observed in the serum of mice that received anti-TNF alone was due mainly to an increase in IgG1 anti-CII-antibodies. In contrast, combination therapy with anti-TNF antibody and anti-IL-12 antibody lowered anti-CII-antibodies of the IgG2a subclass. For this experiment, the number of mice in each treatment group were: control group, n=9; anti-IL-12 antibody alone, n=7; anti-TNF antibody alone, n=8; and anti-TNF antibody plus anti-IL-12 antibody, n=9.

### Lymph Node Cells Ex Vivo and In Vitro

Inguinal lymph node cells (LNC) were removed from treated mice sacrificed at day 10 of arthritis, teased apart to make a single cell suspension, washed and then cultured in 96-well plates at a density of 1x10⁶ cells/ml (200 µl/well) in complete medium comprising RPMI (Bio-Whittaker, Verviers, Belgium), streptomycin (100 µg/ml) (Bio-Whittaker, Verviers, Belgium), and 2-mercaptoethanol (2x10⁻⁵ M). Cells were cultured in the presence or absence of bovine CII (50 µg/ml) in Tris-buffered saline, pH 7 (Mauri *et al., Eur. J. Immunol., 26*:1511-1514 (1996)). Supernatants were collected after 72 hours and stored at -20°C until cytokine measurement. Alternatively, to measure antigen-specific T-cell proliferation, after 72 hours, cells were pulsed with ³H-thymidine (Amersham International, Amersham, UK) for 6 hours, harvested, and assessed for incorporation of radioactivity. To assess the in vitro effects of the antibodies on antigen-specific T-cell proliferation and IFNγ release, lymph node cells from control mice at day 10 of arthritis (DBA/1 mice with induced arthritis, administered PBS) were cultured in the presence of saturating levels, as determined by inhibitory activity in bioassays, of anti-IL-12 (30 µg/ml) and/or anti-TNF (100 µg/ml). IFNγ release was measured by enzyme-linked immunosorbent assay (ELISA). The stimulation index values given were derived from comparisons between cultures containing CII and unstimulated cultures. The expressed results are the mean values of three separate experiments.

The levels of CII-specific T-cell proliferation were reduced in the mice that received a combination of anti-TNF antibody and anti-IL-12 antibody. LNC from mice that received anti-IL-12 antibody alone or anti-TNF antibody alone retained a high level of CII responsiveness (Figure 5A). In addition, in LNC cultures from mice that received either anti-IL-12 antibody alone or anti-TNF antibody alone, there was a reduced stimulation of antigen-specific IFNγ production. Significantly, treatment with the combination of anti-TNF antibody and anti-IL-12 antibody led to a greater suppression of CII-specific IFNγ release by ex vivo LNC (Figure 5B).

When anti-IL-12 antibody and/or anti-TNF antibody were added *in vitro* to LNC from control mice at day 10 of arthritis, it was found that IFNγ release and antigen-specific T-cell proliferation were unaffected by the addition of anti-TNF antibody. However, the inclusion of anti-IL-12 antibody, or the combination of anti-TNF antibody and anti-IL-12 antibody, reduced both IFNγ release and antigen-specific T-cell proliferation (Figures 6A and 6B). No additional effect was observed when the combination of anti-TNF antibody and anti-IL-12 antibody was added.

### In Vitro Cytokine Production By Synovial Membrane Cells

Groups of three mice (n=6 separate experiments) were sacrificed at day 10 of arthritis and the knee joints were removed. Synovial membranes were excised under a dissecting microscope and digested with collagenase A (1 mg/ml) (Boehringer-Mannheim) and DNAase type IV (150 µg/ml) (Sigma, Dorset, UK) at 37°C for 20 minutes, in the presence of polymyxin B (33 µg/ml) (Sigma, Dorset, UK). The synovial membrane cells were then washed extensively and cultured in 96-well plates at a density of 2x10⁶ cells/well (100 µl/well) in complete medium with anti-IL-12 antibody (30 µg/ml) and/or anti-TNF antibody (100 µg/ml), or without antibodies. Supernatants were collected after 24 hours for TNF and IL-12p70 analysis, or at 72 hours for IL-10 and IFNγ analysis and stored at -20°C until measured for cytokines.

For determination of bioactive TNF levels, an assay was performed using the WEHI 164 cell line (Espevik *et al., J. Immunol. Meth., 95*:99-105 (1986)), as previously described in Baker *et al., Eur. J. Immunol., 24*:2040-2048 (1994). The levels of immunoreactive TNF, IFNγ, IL-12p70 and IL-10 were measured by sandwich ELISA. The antibody pairs used were as follows (listed by capture/detection): TNF, rat monoclonal antibody TX3 (obtained from the ATCC, courtesy of Dr. J. Abrams, DNAX, Palo Alto, CA, USA)/rabbit polyclonal antibody (kindly provided by Dr. W. Buurman, University of Limburg, Maastricht, The Netherlands); IL-12, rat monoclonal antibodies 9A5/5C3 (gifts from Dr. D. Presky, Hoffman LaRoche, Nutley, NJ, USA); IL-10, rat monoclonal antibodies 2A5/SXC-1 (obtained from the ATCC, courtesy of Dr. J. Abrams, DNAX, Palo Alto, CA, USA); IFNγ, R4-6A2 (obtained from the ATCC, courtesy of Dr. J. Abrams, DNAX, Palo Alto, CA, USA)/hamster monoclonal antibody 1222-00 (Genzyme Diagnostics, Cambridge, MA, USA). The sensitivity of TNF, IL-12p70 and IFNγ ELISAs was 40 pg/ml. The detection limit for IL-10 was 15 pg/ml.

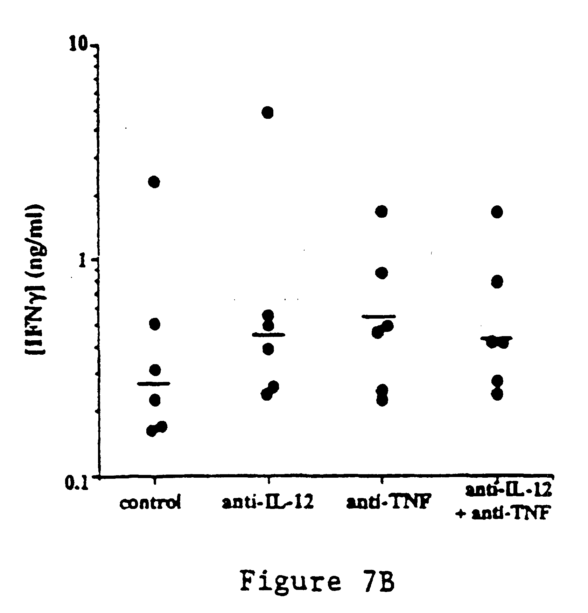

In the primary synovial membrane cell cultures, IL-12p70 was detected in the supernatants in 4 out of 6 experiments (median = 138 pg/ml) (Figure 7A). Anti-IL-12 antibody alone and anti-TNF antibody alone had no effect on IL-12 levels. The combination of anti-IL-12 antibody and anti-TNF antibody was found to have a significant cooperative effect, reducing supernatant levels to below the detection limit. In spite of this effect, there was no subsequent decrease in the levels of IFNγ in the supernatants or cell lysates (Figure 7B). TNF levels in culture supernatants were very low and could not be detected in the ELISA, but could be measured in the WEHI 164 cytotoxicity bioassay and are, thus, expressed as U/ml (Figure 7C). Bioactive TNF levels were not altered by any of the antibody treatment regimens. IL-10, a Th2 cytokine which has anti-inflammatory effects by down-modulating TNF and is an endogenous immunoreaulator in RA (Katsikis *et al., J. Exp. Med., 179*:1517-1527 (1994)), was not detected in control cultures (Figure 7D), but was elevated in the presence of anti-IL-12 antibodies alone, anti-TNF antibodies alone, and the combination of anti-IL-12 antibody and anti-TNF antibody. Median values in Figures 7A-7D are shown by a horizontal bar; dotted lines indicate detection levels.

### Summary

Treatment of CIA with a combination of anti-TNF antibody and anti-IL-12 antibody resulted in dramatic suppression of joint inflammation, as measured by swelling and redness of the hind paws and by decreased spreading of the disease, as reflected in the low clinical scores. Clinical results were confirmed by the histological data, which showed a marked protection against joint destruction in the mice treated with a combination of anti-TNF antibody and anti-IL-12 antibody. This was demonstrated not only in the hind paws but also in the knee joints.

Amelioration of arthritis was not accompanied by changes in total IgG anti-CII antibody levels or in the serum IgG2a/IgG1 ratio after 10 days of arthritis. However, at 20 days of arthritis, there was a reduction in anti-CII IgG2a:IgG1 ratio.

LNC proliferation and IFNγ production were all decreased by combined *in vitro* treatment with anti-IL-12 antibody and anti-TNF antibody of LNC from control mice at day 10 of arthritis (DBA/1 mice with induced arthritis, administered PBS).

In synovial cell cultures, IL-12 was decreased and IL-10 production was increased by the combination of anti-IL-12 antibody and anti-TNF antibody.

The magnitude of the benefit obtained by combining anti-IL-12 and anti-TNF antibodies was considerable, unlike treatment with anti-IL-12 antibody alone. The results show a synergy between anti-TNF antibody and anti-IL-12 antibody in blocking progression of CIA after onset of disease.
This suggests that therapy with a combination of anti-TNF antibody and anti-IL-12 antibody is valuable in the treatment of rheumatoid arthritis.

### EQUIVALENTS

Those skilled in the art will know, or be able to ascertain, using no more than routine experimentation, many equivalents to the specific embodiments of the invention described herein. These and all other equivalents are intended to be encompassed by the following claims.

## Claims

1. A combined preparation comprising a tumor necrosis factor antagonist and an interleukin-12 antagonist.

2. The combined preparation of claim 1, wherein the interleukin-12 antagonist is an anti-interleukin-12 antibody or antigen-binding fragment thereof.

3. The combined preparation of claim 1 or claim 2, wherein the tumor necrosis factor antagonist is an anti-tumor necrosis factor antibody or chimeric antibody, or antigen-binding fragment thereof.

4. A combined preparation as claimed in claim 1, 2 or 3 for simultaneous, separate or sequential use in treating or preventing a tumor necrosis factor-α-mediated disease in an individual.

5. The combined preparation of claim 4, wherein the tumor necrosis factor-α-mediated disease is selected from the group consisting of autoimmune disease, acute or chronic immune disease, inflammatory disease and neurodegenerative disease.

6. The combined preparation of claim 4 or claim 5, wherein the tumor necrosis factor antagonist prevents or inhibits tumor necrosis factor synthesis, tumor necrosis factor release or tumor necrosis factor action on target cells.

7. The combined preparation of claim 6, wherein the tumor necrosis factor antagonist is selected from the group consisting of an anti-tumor necrosis factor antibody, a chimeric antibody or antigen-binding fragment thereof, a tumor necrosis factor receptor/immunoglobulin G fusion protein, a phosphodiesterase inhibitor and thalidomide.

8. The combined preparation of claim 7, wherein the phosphodiesterase inhibitor is selected from the group consisting of rolipram and pentoxifylline.

9. A combined preparation of claim 1 in therapeutically effective amounts, for treating or preventing a disease selected from the group consisting of rheumatoid arthritis, Crohn's disease and acute or chronic immune disease associated with a transplantation in an individual.

10. The combined preparation of claim 9, wherein the transplantation is selected from the group consisting of renal transplantation, cardiac transplantation, bone marrow transplantation, liver transplantation, pancreatic transplantation, small intestine transplantation, skin transplantation and lung transplantation.

11. The combined preparation of claim 9 or claim 10, wherein the tumor necrosis factor antagonist is an anti-tumor necrosis factor antibody or chimeric antibody, or antigen-binding fragment thereof, and/or the interleukin-12 antagonist is an anti-interleukin-12 antibody or antigen-binding fragment thereof.

12. The combined preparation of claim 4 or claim 9, wherein the tumor necrosis factor antagonist is a phosphodiesterase inhibitor.

13. The combined preparation of claim 12, wherein the phosphodiesterase inhibitor is rolipram.

14. The combined preparation of claim 4 or claim 9, wherein the disease is rheumatoid arthritis, the interleukin-12 antagonist is an anti-interleukin-12 antibody or antigen-binding fragment thereof and the tumor necrosis factor antagonist is rolipram.

15. Use of a tumor necrosis factor antagonist and an interleukin-12 antagonist for the manufacture of a medicament or prophylactic agent for use against tumor necrosis factor-α-mediated disease, wherein the tumor necrosis factor-α-mediated disease is selected from the group consisting of rheumatoid arthritis, Crohn's disease and acute or chronic immune diseases associated with transplantation.

16. The use of claim 15, wherein the combined preparations of any of claims 1 to 14 are used.

17. The use of claim 15, wherein the tumor necrosis factor antagonist is selected from the group consisting of a tumor necrosis factor receptor-immunoglobulin G fusion protein and a phosphodiesterase inhibitor.

18. The use of claim 17, wherein the phosphodiesterase inhibitor is selected from the group consisting of pentoxifylline and rolipram.

19. Use of a tumor necrosis factor antagonist for the manufacture of a medicament or prophylactic agent for use with an interleukin-12 antagonist in the treatment of tumor necrosis factor-α-mediated disease, wherein the tumor necrosis factor-α-mediated disease is selected from the group consisting of rheumatoid arthritis, Crohn's disease and acute or chronic immune diseases associated with transplantation.

20. Use of an interleukin-12 antagonist for the manufacture of a medicament or prophylactic agent for use with a tumor necrosis factor antagonist in the treatment of tumor necrosis factor-α-mediated disease, wherein the tumor necrosis factor-α-mediated disease is selected from the group consisting of rheumatoid arthritis, Crohn's disease and acute or chronic immune diseases associated with transplantation.

## Patentansprüche

1. Kombipräparat mit einem Tumornekrosefaktor-Antagonisten und einem Interleukin-12-Antagonisten.

2. Kombipräparat nach Anspruch 1, bei dem der Interleukin-12-Antagonist ein Anti-Interleukin-12-Antikörper oder ein Antigen-bindendes Fragment von diesem ist.

3. Kombipräparat nach Anspruch 1 oder 2, bei dem der Tumornekrosefaktor-Antagonist ein Antitumornekrosefaktor-Antikörper oder Chimären-Antikörper oder davon ein Antigen-bindendes Fragment ist.

4. Kombipräparat nach Anspruch 1, 2 oder 3 für eine simultane, separate oder sequentielle Verwendung bei der Behandlung oder Verhinderung einer durch den Tumornekrosefaktor α vermittelten Krankheit bei einem Individuum.

5. Kombipräparat nach Anspruch 4, bei dem die durch den Tumornekrosefaktor α vermittelte Krankheit ausgewählt ist aus der aus Autoimmunkrankheit, akuter oder chronischer Immunkrankheit, Entzündungskrankheit und neurodegenerativer Krankheit bestehenden Gruppe.

6. Kombipräparat nach Anspruch 4 oder 5, bei dem der Tumornekrosefaktor-Antagonist eine Tumornekrosefaktor-Synthese, ein Tumornekrosefaktor-Release oder eine Tumornekrosefaktor-Aktion bei Target-Zellen verhindert.

7. Kombipräparat nach Anspruch 6, bei dem der Tumornekrosefaktor-Antagonist ausgewählt ist aus der aus einem Antitumornekrosefaktor-Antikörper, einem Chimären-Antikörper oder davon einem Antigen-bindenden Fragment, einem Tumornekrosefaktor-Rezeptor/Immunglobin-G-Fusionsprotein, einem Phosphodiesterasehemmer und Thalidomid bestehenden Gruppe.

8. Kombipräparat nach Anspruch 7, bei dem der Phospodiesterasehemmer ausgewählt ist aus der aus Rolipram und Pentoxifyllin bestehenden Gruppe.

9. Kombipräparat nach Anspruch 1 in therapeutisch wirksamen Mengen zur Behandlung oder Verhinderung einer Krankheit aus der aus rheumatoider Arthritis, Crohn-Krankheit und akuter oder chronischer Immunkrankheit in Verbindung mit einer Transplantation bei einem Individuum bestehenden Gruppe.

10. Kombipräparat nach Anspruch 9, bei dem die Transplantation ausgewählt ist aus der aus Nierentransplantation, Herztransplantation, Knochenmarktransplantation, Lebertransplantation, Bauspeicheldrüsentransplantation, Dünndarmtransplantation, Hauttransplantation und Lungentransplantation bestehenden Gruppe.

11. Kombipräparat nach Anspruch 9 oder 10, bei dem der Tumomekrosefaktor-Antagonist ein Antitumornekrosefaktor-Antikörper oder Chimären-Antikörper oder davon ein Antigen-bindendes Fragment und/oder der Interleukin-12-Antagonist ein Anti-Interleukin-12-Antikörper oder davon ein Antigen-bindendes Fragment ist.

12. Kombipräparat nach Anspruch 4 oder 9, bei dem der Tumornekrosefaktor-Antagonist ein Phosphodiesterasehemmer ist.

13. Kombipräparat nach Anspruch 12, bei dem der Phosphodiesterasehemmer Rolipram ist.

14. Kombipräparat nach Anspruch 4 oder 9, bei dem die Krankheit rheumatoide Arthritis ist, der Interleukin-12-Antagonist ein Anti-Interleukin-12-Antikörper oder davon ein Antigen-bindendes Fragment und der Tumornekrosefaktor-Antagonist Rolipram ist.

15. Verwendung eines Tumornekrosefaktor-Antagonisten und eines Interleukin-12-Antagonisten für die Herstellung eines Medikamentes oder prophylaktischen Mittels zur Verwendung gegen durch den Tumornekrosefaktor α vermittelte Krankheit, wobei die durch den Tumornekrosefaktor α vermittelte Krankheit ausgewählt ist aus der aus rheumatoider Arthritis, Crohn-Krankheit und akuten oder chronischen Immunkrankheiten in Verbindung mit einer Transplantation bestehenden Gruppe.

16. Verwendung nach Anspruch 15, bei der die Kombipräparate nach einem der Ansprüche 1 bis 14 verwendet werden.

17. Verwendung nach Anspruch 15, bei der der Tumornekrosefaktor-Antagonist ausgewählt ist aus der aus einem Tumornekrosefaktor-Rezeptor/Immunglobulin-G-Fusionsprotein und einem Phosphodiesterasehemmer bestehenden Gruppe.

18. Verwendung nach Anspruch 17, bei der der Phosphodiesterasehemmer ausgewählt ist aus der aus Pentoxifyllin und Rolipram bestehenden Gruppe.

19. Verwendung eines Tumornekrosefaktor-Antagonisten für die Herstellung eines Medikaments oder prophylaktischen Mittels zur Verwendung mit einem Interleukin-12-Antagonisten bei der Behandlung einer durch den Tumornekrosefaktor α vermittelten Krankheit, wobei die durch den Tumornekrosefaktor α vermittelte Krankheit ausgewählt ist aus der aus rheumatoider Arthritis, Crohn-Krankheit und akuten oder chronischen Immunkrankheiten in Verbindung mit einer Transplantation bestehenden Gruppe.

20. Verwendung eines Interleukin-12-Antagonisten für die Herstellung eines Medikaments oder prophylaktischen Mittels für eine Verwendung mit einem Tumornekrosefaktor-Antagonisten bei der Behandlung einer durch den Tumornekrosefaktor α vermittelten Krankheit, wobei die durch den Tumornekrosefaktor α vermittelte Krankheit ausgewählt ist aus der aus rheumatoider Arthritis, Crohn-Krankheit und akuten oder chronischen Immunkrankheiten in Verbindung mit einer Transplantation bestehenden Gruppe.

## Revendications

1. Préparation combinée qui comprend un antagoniste d'un facteur de nécrose des tumeurs et un antagoniste d'interleukine-12.

2. Préparation combinée selon la revendication 1, dans laquelle l'antagoniste de l'interleukine-12 est un anticorps anti-interleukine-12 ou un fragment de celui-ci qui se lie à l'antigène.

3. Préparation combinée selon la revendication 1 ou la revendication 2, dans laquelle l'antagoniste du facteur de nécrose des tumeurs est un anticorps anti-facteur de nécrose des tumeurs, un anticorps chimérique ou un fragment de ces anticorps qui se lie à l'antigène.

4. Préparation combinée selon les revendications 1, 2 ou 3, destinée à l'utilisation simultanée, séparée ou successive dans le traitement ou la prévention chez un individu d'une maladie α-médiée par le facteur de nécrose des tumeurs.

5. Préparation combinée selon la revendication 4, dans laquelle la maladie α-médiée par le facteur de nécrose des tumeurs est sélectionnée dans le groupe constitué des maladies auto-immunes, des maladies immunes aiguës ou chroniques, des maladies inflammatoires et des maladies neurodégénérescentes.

6. Préparation combinée selon la revendication 4 ou la revendication 5, dans laquelle l'antagoniste du facteur de nécrose des tumeurs empêche ou inhibe la synthèse du facteur de nécrose des tumeurs, la libération du facteur de nécrose des tumeurs ou l'action du facteur de nécrose des tumeurs sur les cellules cibles.

7. Préparation combinée selon la revendication 6, dans laquelle l'antagoniste du facteur de nécrose des tumeurs est choisi dans le groupe constitué d'un anticorps anti-facteur de nécrose des tumeurs, d'un anticorps chimérique ou d'un fragment de ces anticorps qui se lie à l'antigène, une protéine de fusion du récepteur du facteur de nécrose des tumeurs et d'immunoglobuline G, d'un inhibiteur de phosphodiestérase et de la thalidomide.

8. Préparation combinée selon la revendication 7, dans laquelle l'inhibiteur de la phosphodiestérase est choisi dans le groupe constitué du rolipram et de la pentoxyphylline.

9. Préparation combinée selon la revendication 1, en quantité thérapeutiquement efficace pour le traitement ou la prévention d'une maladie sélectionnée dans le groupe constitué de l'arthrite rhumatoïde, de la maladie de Crohn et d'une maladie immune aiguë ou chronique associée à une transplantation chez un individu.

10. Préparation combinée selon la revendication 9, dans laquelle la transplantation est sélectionnée dans le groupe constitué de la transplantation rénale, la transplantation cardiaque, la transplantation de moelle épinière, la transplantation du foie, la transplantation du pancréas, la transplantation de l'intestin grêle, la transplantation de la peau et la transplantation des poumons.

11. Préparation combinée selon la revendication 9 ou la revendication 10, dans laquelle l'antagoniste du facteur de nécrose des tumeurs est un anticorps anti-facteur de nécrose des tumeurs, un anticorps chimérique ou un fragment de ces anticorps qui se lie à l'antigène, et/ou l'antagoniste de l'interleukine-12 est un anticorps anti-interleukine-12 ou un fragment de cet anticorps qui se lie à l'antigène.

12. Préparation combinée selon la revendication 4 ou la revendication 9, dans laquelle l'antagoniste du facteur de nécrose des tumeurs est un inhibiteur de la phosphodiestérase.

13. Préparation combinée selon la revendication 12, dans laquelle l'inhibiteur de la phosphodiestérase est le rolipram.

14. Préparation combinée selon la revendication 4 ou la revendication 9, dans laquelle la maladie est l'arthrite rhumatoïde, l'antagoniste de l'interleukine-12 est un anticorps anti-interleukine-12 ou un fragment de cet anticorps qui se lie à l'antigène, l'antagoniste du facteur de nécrose des tumeurs étant le rolipram.

15. Utilisation d'un antagoniste du facteur de nécrose des tumeurs et d'un antagoniste de l'interleukine-12 pour la fabrication d'un médicament ou d'un agent prophylactique destiné à être utilisé contre des maladies α-médiées par le facteur de nécrose des tumeurs, la maladie α-médiée par le facteur de nécrose des tumeurs étant sélectionnée dans le groupe constitué de l'arthrite rhumatoïde, de la maladie de Crohn et des maladies immunes aiguës ou chroniques associées à une transplantation.

16. Utilisation selon la revendication 15, dans laquelle on utilise les préparations selon l'une quelconque des revendications 1 à 14.

17. Utilisation selon la revendication 15, dans laquelle l'antagoniste du facteur de nécrose des tumeurs est sélectionné dans le groupe constitué d'une protéine qui résulte de la fusion d'un récepteur du facteur de nécrose des tumeurs et d'immunoglobuline G ainsi que d'un inhibiteur de la phosphodiestérase.

18. Utilisation selon la revendication 17, dans laquelle l'inhibiteur de la phosphodiestérase est sélectionné dans le groupe constitué de la pentoxyphylline et du rolipram.

19. Utilisation d'un antagoniste du facteur de nécrose des tumeurs pour la fabrication d'un médicament ou d'un agent prophylactique destiné à être utilisé avec un antagoniste de l'interleukine-12 dans le traitement de maladies α-médiées par le facteur de nécrose des tumeurs, la maladie α-médiée par le facteur de nécrose des tumeurs étant sélectionnée dans le groupe constitué de l'arthrite rhumatoïde, de la maladie de Crohn et des maladies immunes aiguës ou chroniques associées à une transplantation.

20. Utilisation d'un antagoniste de l'interleukine-12 pour la fabrication d'un médicament ou d'un agent prophylactique destiné à être utilisé avec un antagoniste du facteur de nécrose des tumeurs dans le traitement de maladies α-médiées par le facteur de nécrose des tumeurs, la maladie α-médiée par le facteur de nécrose des tumeurs étant choisie dans le groupe constitué de l'arthrite rhumatoïde, de la maladie de Crohn et des maladies immunes aiguës ou chroniques associées à une transplantation.
